Europäisches Patentamt

European Patent Office

Office européen des brevets

(19)

(11) Numéro de publication: **0 283 338 B1**

(12) **FASCICULE DE BREVET EUROPEEN**

(45) Date de publication de fascicule du brevet: **28.09.94**

(51) Int. Cl.5: **C12N 15/29**, C12N 15/53,
//C12N9/06

(21) Numéro de dépôt: **88400330.2**

(22) Date de dépôt: **15.02.88**

(54) **Vecteur de clonage du gène de structure de la nitrate réductase.**

(30) Priorité: **16.02.87 FR 8701925**
**27.07.87 FR 8710621**

(43) Date de publication de la demande:
**21.09.88 Bulletin 88/38**

(45) Mention de la délivrance du brevet:
**28.09.94 Bulletin 94/39**

(84) Etats contractants désignés:
**AT BE CH DE ES FR GB GR IT LI LU NL SE**

(56) Documents cités:

**J. CELL. BIOCHEM. SUPPL. 1987, vol. 0, no. 11, partie B, page 24& 16TH ANNUAL UCLA SYMPOSIUM ON PLANT GENE SYSTEMS ANDTHEIR BIOLOGY, 2-8 février 1987; M. VINCENTZ et al.: "Cloning of DNA fragments complementary to tobacco nitrate reductase mRNA and encoding epitopes common to the nitrate reductases from higher plants"**

(73) Titulaire: **INSTITUT NATIONAL DE LA RECHERCHE AGRONOMIOUE**
**145, rue de l'Université**
**F-75341 Paris Cédex 07 (FR)**

(72) Inventeur: **Huttener, Eric**
**2, rue de la Durance**
**F-75012 Paris (FR)**
Inventeur: **Calza, Roger**
**24, les Nouveaux Horizons**
**F-78310 Elancourt (FR)**
Inventeur: **Caboche, Michel**
**5, rue du Thinerais**
**F-78310 Maurepas (FR)**
Inventeur: **Vaucheret, Hervé**
**15, rue Léon Bloy**
**F-92260 Fontenay-Aux-Roses (FR)**

(74) Mandataire: **Warcoin, Jacques et al**
**Cabinet Régimbeau**
**26, avenue Kléber**
**F-75116 Paris (FR)**

Rank Xerox (UK) Business Services
(3.10/3.09/3.3.3)

PROCEEDINGS OF THE NAT. ACADEMY OF SCIENCES OF USA, vol. 83, no. 21, novembre1986, pages 8073-8076, Washington, DC, US; N.M. CRAWFORD et al.: "Nitrate reductase from squash: cDNA cloning and nitrate regulation"

PROCEEDINGS OF THE NAT. ACADEMY OF SCIENCES OF USA, vol. 83, septembre 1986,pages 6825-6828, Washington, DC, US; C.-L. CHENG et al.: "Cloning and nitrate induction of nitrate reductase mRNA"

SCIENCE, vol. 233, 25 juillet 1986, pages 478-481; D.M. SHAH et al.:"Engineering herbicide tolerance in transgenic plants"

MOL. GEN. GENET, vol. 209, 1987, pages 552-562, Springer-Verlag; R. CALZA etal.: "Cloning of DNA fragments complementary to tobacco nitrate reductase mRNA and encoding epitopes common to the nitrate reductases from higher plants"

Plant Science 44 (1986) pp. 191-203

## Description

Le domaine technique de la présente invention est celui de l'isolement de gènes de structure de protéines à partir d'espèces végétales. Plus particulièrement, l'invention concerne le clonage de fragments d'ADN complémentaire de l'ARNm de la nitrate réductase de tabac et codant pour des épitopes communs aux nitrates réductases (NR) des plantes supérieures. L'invention concerne donc également les clones obtenus, l'ADNc isolé selon l'invention codant pour plus de 50 % de l'ARNm de la NR de tabac.

La présente invention concerne également des vecteurs de clonage comportant un fragments d'ADNᶜ de l'ARNm de la nitrate réductase de tabac comprenant la séquence codante de nucléotides partielle ou complète.

La nitrate réductase (NR) est une enzyme clé qui rentre en jeu dans la première étape de l'assimilation du nitrate dans les plantes. Or, l'assimilation des nitrates constitue une étape limitante du développement des plantes. Par conséquent, le clonage du gène de structure de la NR et sa réintroduction dans une plante est du plus haut intérêt pour modifier les caractéristiques d'assimilation des nitrates par les plantes. Une majorité des plantes assimilent, par exemple, exclusivement au niveau foliaire. Mais il serait avantageux, pour certaines plantes, d'assimiler les nitrates au niveau racinaire, en particulier pour les espèces stockant leurs reserves dans les racines ou tubercules telles que la pomme de terre. En revanche, certaines espèces ligneuses, comme les arbres fruitiers, assimilent beaucoup de nitrates au niveau racinaire et leur assimilation foliaire serait mieux adaptée à la fructification.

Chez les monocotylédones, les nitrates doivent être apportés à des stades critiques du développement, ce qui suggère que l'assimilation des nitrates n'est pas effectuée de façon uniforme dans le temps chez ces plantes. Certaines espèces ne semblent plus assimiler les nitrates lorsque les graines commencent à se développer, épuisant ainsi les réserves en azote réduit foliaire. Introduire des gènes de nitrate réductase dont "la mise à feu" sera différente de celle observée pour le gène résidant permettra d'obtenir des plantes ayant des caractéristiques de culture et de production améliorées.

Dans le domaine de l'enzymologie (enzymes fixées), la nitrate réductase est une protéine qui porte dans sa structure quatre sites d'attachement de cofacteurs : NADH, FAD, cytochrome $b_{557}$, cofacteur à molybdène (MoCo). L'obtention de fragments polypeptidiques correspondant à une partie de l'enzyme (synthétisée dans E. coli à partir de fragments d'ADNc grâce à l'emploi d'un vecteur d'expression) permet d'envisager la mise au point de colonnes d'affinité pour ces molécules. La nitrate réductase catalyse une série d'étapes d'oxydoréduction (NADH $\rightleftharpoons$ NAD$^+$ ; FADH $\rightleftharpoons$ FAD$^+$) ; cyt b557 red $\rightleftharpoons$ cyt b557 ox ; MoCo red $\rightleftharpoons$ MoCo ox ; NO$_3^-$ $\rightleftharpoons$ NO$_2^-$ ; Fe$^{3+}$ $\rightleftharpoons$ Fe$^{2+}$). Ceci peut être utilisé, par exemple, pour éliminer les nitrites toxiques en les convertissant en nitrates, et aussi pour mesurer les concentrations de ces molécules grâce à son emploi dans des capteurs à sonde protéique (électrode à enzyme).

Dans le domaine des cultures cellulaires, certaines lignées cellulaires ne produisent de métabolites secondaires que dans certaines conditions de croissance particulières (carence en sucres ou en source azotée). L'introduction de gènes de NR à expression contrôlée dans des cellules récipients NR$^-$ obtenues par mutagénèse permettra d'induire à volonté une carence éventuelle en source azotée grâce à l'emploi de promoteurs adaptés et de favoriser ainsi la production de l'alcaloîde désiré.

C'est pourquoi la présente invention concerne plus particulièrement un vecteur de clonage comportant un ADN complémentaire de l'ARNm de la nitrate réductase de tabac.

Parmi ces vecteurs, il faut citer les vecteurs comportant un ADN complémentaire de l'ARNm de la nitrate réductase de tabac comportant, sur un fragment EcoRI/EcoRI de cet ADNc, dans l'ordre, les sites suivants : XmnI, HindIII, KpnI, EcoRI, ou KpnI, HindIII, XmnI, EcoRI.

Ces vecteurs sont, de préférence, des vecteurs constitué d'un phage dans lequel est inséré l'ADNc.

Plus particulièrement, les ADNc isolés dans les exemples représentent plus de 50 % de l'ARNm de la nitrate réductase de tabac. Les inserts d'ADNc ont, en général, plus de 1,6 kb.

La présente invention a également pour objet un vecteur de clonage comportant un fragment d'ADNc de l'ARNm de la nitrate réductase comportant la séquence codante de nucléotides suivante :

```
GAA TTC CCT TGT AGA GAA TTG CCC GTT ACG CTT GTT TGT GCT CGC AAT CGA AGG AAA GAA CAG
AAC ATG GTT AAA CAA ACC ATT GGT TTC AAC TCG CGC GCC GCT GCC GTT TCA ACA ACG ATA TGG
CGC GGG GTA CCC CTC CGC GCT TTG CTA AAA CGG TGC GGT GTT TTT AGC AAG AAT AAA GCG GCG
CTT AAT GTT TGC TTC GAA GGA GCT GAT GTG TTG CCC GGA GGT GGT GGT TCA AAG TAT GGA ACC
AGC ATT AAG AAG GAA TTT CCA ATG GAT CCA GCA CGA GAT ATC ATC GTA GCC TAC ATG CAG AAC
GGA GAA AAA TTG GCA CCC GAC CAC GGG TTT CCA GTA CGA ATG ATA ATT CCA GGA TTC ATT GGA
GGA AGA ATG GTG AAA TGG ATA AAG AGG ATT ATA GTC ACC ACC CAA GAA TCA GAC AGC TAT TAT
CAT TTC AAG CAC AAT AGA GTT CTT CCT CCC CAT GTT GAT GCT GAA CTT GCA AAT ACC GAA GCA
TGG TGG TAC AAG CCA GAG TAT ATC ATC AAT CAG CTT AAT ATT AAC TCT GTC ATT ACG ACG CCG
TGT CAT GAA GAA ATT TTG CCA ATT AAC GCC TGG ACG ACT CAG CGA CCT TAC ACG TTG AGG GGC
TAT TCT TAT TCT GGC GGA GGG AAA AAA GTA ACG CGA GTA GAA GTG ACG TTG GAT GGA GGA GAA
ACA TGG CAA GTT AGC ACA CTA GAT CAC CCA GAG AAG CCC ACC AAA TAT GGC AAG TAC TGG TGT
TGG TGC TTT TGG TCA CTC GAG GTT GAG GTG TTA GAC TTG CTC AGT GCT AAA GAA ATT GCT GTT
CGA GCT TGG GAT GAG ACC CTC AAT ACT CAA CCC GAG AAG CTT ATT TGG AAC GTC ATG GGA ATG
ATG AAT AAT TGC TGG TTC CGA GTA AAG ATG AAT GTG TGC AAG CCT CAC AAG GGA GAG ATT GGA
ATA GTG TTT GAG CAT CCG ACT CAA CCT GGA AAC CAA TCA GGT GGA TGG ATG GCG AAG GAG AGA
CAT TTG GAG ATA TCA GCA GAG GCA CCT CAA ACA CTA AAG AAG AGT ATC TCA ACT CCA TTC ATG
AAC ACA GCT TCC AAG ATG TAC TCC ATG TCC GAG GTC AGG AAA CAC AGC TCT GCT GAC TCT GCT
TGG ATC ATA GTC CAT GGT CAT ATC TAT GAC GCC ACG CGT TTC TTG AAA GAT CAC CCT GGT GGG
ACT GAC AGC ATT CTC ATC AAT GCT GGC ACT GAT TGC ACT GAG GAA TTT GAT GCA ATT CAT TCT
GAT AAG GCT AAG AAG CTC TTG GAG GAT TTC AGG ATT GGT GAA CTC ATA ACT ACT GGT TAC ACC
TCT GAC TCT CCT GGC AAC TCC GTG CAC GGA TCT TCT TCC TTC AGC AGC TTT CTA GCA CCT ATT
AAG GAA CTT GTT CCA GCG CAG AGG AGT GTG GCC CTA ATT CCA AGA GAG AAA ATC CCA TGC AAA
CTC ATC GAC AAG CAA TCC ATC TCC CAT GAT GTT AGG AAA TTT CGA TTT GCA TTG CCC TCT GAG
GAT CAA GTC TTG GGC TTG CCT GTT GGA AAA CAT ATC TTC CTC TGT GCC GTT ATT GAC GAT AAG
CTC TGC ATG CGC GCT TAC ACG CCT ACT AGC ACG ATC GAT GAG GTG GCG TAC TTC GAG TTG GTT
GTC AAG ATA TAC TTC AAA GGA ATT
```

Enfin, la présente invention a également pour objet un vecteur de clonage comportant un fragment d'ADNc de l'ARNm de la nitrate réductase de tabac comprenant la séquence complète de nucléotides suivante :

```
                        ATG GCG GCA TCT GTC GAA AAC AGG CAG

TTC AGT CAC CTA GAA GCC GGT TTA TCC CGG TCT TTC AAG CCC

CGG TCT GAT TCC CCG GTT CGT GGC TGC AAC TTC CCT TCG CCC

AAC AGT ACT AAT TTC CAA AAG AAA CCA AAT TCC ACC ATT TAC

CTT GAT TAC TCG TCG AGT GAA GAC GAC GAT GAT GAT GAC GAA

AAA AAT GAG TAC CTT CAA ATG ATT AAA AAA GGG AAT TCA GAG

TTA GAG CCA TCT GTT CAT GAC ACT AGG GAC GAA GGT ACC GCT

GAT AAT TGG ATT GAA CGC AAC TTT TCC ATG ATT CGT CTC ACC

GGA AAG CAT CCA TTT AAC TCC GAA CCA CCG TTG AAC CGG CTC

ATG CAC CAC GGC TTT ATC ACA CCG GTC CCA CTT CAT TAC GTT

CGT AAC CAT GGA CCG GTT CCC AAG GGC ACG TGG GAT GAC TGG

ACC GTG GAA GTC ACG GGA CTA GTG AAG CGT CCT ATG AAA TTC

ACA ATG GAC CAG TTG GTT AAC GAA TTC CCT TGT AGA GAA TTG

CCC GTT ACG CTT GTT TGT GCT GGC AAT CGA AGG AAA GAA CAG

AAC ATG GTT AAA CAA ACC ATT GGT TTC AAC TGG GGC GCC GCT

GCC GTT TCA ACA ACG ATA TGG CGC GGG GTA CCC CTC CGC GCT

TTG CTA AAA CGG TGC GGT GTT TTT AGC AAG AAT AAA GGG GCG

CTT AAT GTT TGC TTC GAA GGA GCT GAT GTG TTG CCC GGA GGT

GGT GGT TCA AAG TAT GGA ACC AGC ATT AAG AAG GAA TTT GCA

ATG GAT CCA GCA CGA GAT ATC ATC GTA GCC TAC ATG CAG AAC

GGA GAA AAA TTG GCA CCC GAC CAC GGG TTT CCA GTA CGA ATG

ATA ATT CCA GGA TTC ATT GGA GGA AGA ATG GTG AAA TGG ATA

AAG AGG ATT ATA GTC ACC ACC CAA GAA TCA GAC AGC TAT TAT

CAT TTC AAG GAC AAT AGA GTT CTT CCT CCC CAT GTT GAT GCT

GAA CTT GCA AAT ACC GAA GCA TGG TGG TAC AAG CCA GAG TAT

ATC ATC AAT GAG CTT AAT ATT AAC TCT GTC ATT ACG ACG CCG

TGT CAT GAA GAA ATT TTG CCA ATT AAC GCC TGG ACG ACT CAG

CGA CCT TAC ACG TTG AGG GGC TAT TCT TAT TCT GGC GGA GGG

AAA AAA GTA ACG CGA GTA GAA GTG ACG TTG GAT GGA GGA GAA

ACA TGG CAA GTT AGC ACA CTA GAT CAC CCA GAG AAG CCC ACC

AAA TAT GGC AAG TAC TGG TGT TGG TGC TTT TGG TCA CTC GAG

GTT GAG GTG TTA GAC TTG CTC AGT GCT AAA GAA ATT GCT GTT

CGA GCT TGG GAT GAG ACC CTC AAT ACT CAA CCC GAG AAG CTT
```

```
ATT TGG AAC GTC ATG GGA ATG ATG AAT AAT TGC TGG TTC CGA
GTA AAG ATG AAT GTG TGC AAG CCT CAC AAG GGA GAG ATT GGA
ATA GTG TTT GAG CAT CCG ACT CAA CCT GGA AAC CAA TCA GGT
GGA TGG ATG GCG AAG GAG AGA CAT TTG GAG ATA TCA GCA GAG
GCA CCT CAA ACA CTA AAG AAG AGT ATC TCA ACT CCA TTC ATG
AAC ACA GCT TCC AAG ATG TAC TCC ATG TCC GAG GTC AGG AAA
CAC AGC TCT GCT GAC TCT GCT TGG ATC ATA GTC CAT GGT CAT
ATC TAT GAC GCC ACG CGT TTC TTG AAA GAT CAC CCT GGT GGG
ACT GAC AGC ATT CTC ATC AAT GCT GGC ACT GAT TGC ACT GAG
GAA TTT GAT GCA ATT CAT TCT GAT AAG GCT AAG AAG CTC TTG
GAG GAT TTC AGG ATT GGT GAA CTC ATA ACT ACT GGT TAC ACC
TCT GAC TCT CCT GGC AAC TCC GTG CAC GGA TCT TCT TCC TTC
AGC AGC TTT CTA GCA CCT ATT AAG GAA CTT GTT CCA GCG CAG
AGG AGT GTG GCC CTA ATT CCA AGA GAG AAA ATC CCA TGC AAA
CTC ATC GAC AAG CAA TCC ATC TCC CAT GAT GTT AGG AAA TTT
CGA TTT GCA TTG CCC TCT GAG GAT CAA GTC TTG GGC TTG CCT
GTT GGA AAA CAT ATC TTC CTC TGT GCC GTT ATT GAC GAT AAG
CTC TGC ATG CGC GCT TAC ACG CCT ACT AGC ACG ATC GAT GAG
GTG GGG TAC TTC GAG TTG GTT GTC AAG ATA TAC TTC AAA GGA
ATT CAC CCT AAA TTC CCC AAT GGA GGG CAA ATG TCA CAG TAT
CTT GAT TCT ATG CCG TTA GGG TCA TTT CTC GAC GTG AAA GGT
CCA TTA GGT CAC ATT GAA TAC CAA GGA AAG GGA AAT TTC TTA
GTT CAT GGC AAA CAG AAG TTT GCC AAG AAG TTG GCC ATG ATA
GCA GGT GGA ACA GGA ATA ACT CCA GTG TAT CAA GTC ATG CAG
GCA ATT CTG AAA GAT CCA GAA GAT GAC ACA GAA ATG TAT GTG
GTG TAT GCT AAC AGA ACA GAG GAT GAT ATT TTA CTT AAG GAA
GAG CTT GAT TCA TGG GCT GAG AAA ATT CCA GAG AGG GTT AAA
GTT TGG TAT GTG GTT CAG GAT TCT ATT AAA GAA GGA TGG AAG
TAC AGC ATT GGT TTT ATT ACA GAA GCC ATT TTG AGA GAA CAT
ATC CCT GAG CCA TCT CAC ACA ACA CTG GCT TTG GCT TGT GGA
CCA CCT CCT ATG ATT CAA TTT GCT GTT AAT CCA AAC TTG GAG
AAG ATG GGC TAT GAC ATT AAG GAT TCC TTA TTG GTG TTC TAA
```

La présente invention a en outre pour objet les sondes d'ADNc de l'ARNm de la nitrate réductase de tabac comportant les séquences codantes de nucléotides ci-dessus.

Ces sondes peuvent être exploitées notamment pour l'isolement du gène correspondant qui permet de créer des plants différents dans leurs caractéristiques d'assimilation des nitrates.

L'invention sera maintenant mieux comprise à la lumière de la description détaillée qui va suivre. Celle-ci comprend notamment une série d'exemples. Elle est faite en référence à des figures dans lesquelles sont présentés :

- figure 1 : la traduction acellulaire dans un système réticulocyte de l'ARN messager codant pour l'apoenzyme NR ; les ARNm PolyA$^+$ ont été isolés de jeunes feuilles de tabac et traduits en dehors de la cellule ; les produits de translation ont été fractionnés sur gel dénaturant polyacrylamide 5 %.
- figure 2 : le criblage immunologique d'une banque d'ADNc de tabac.
- figure 3 : le diagramme d'une famille de clones s'hybridant avec le fragment EcoRI de 1,6 kb.
- figure 4 : une analyse de "western blot" des protéines de fusion synthétisées par divers lysogènes.
- figure 5 : l'inhibition de l'activité catalytique de la NR de tabac par des immunoglobulines immunosélectionnées par adsorption sur des plaques formées par des phages lambdaGT11 et 13-29 en présence d'IPTG.
- figure 6 : une analyse de "northern blot" des séquences d'ARNm s'hybridant avec le fragment EcoRI de 1,6 kb.
- figure 7 : une analyse de "southern blot" des séquences génomiques de tabac homologues du fragment EcoRI de 1,6 kb.
- Les figures 8a et 8b représentent une séquence partielle de nucléotides de l'insert d'ADNc du phage recombinant lambda 13-29, et la séquence d'aminoacides correspondante de la partie d'ADNc codée de la protéine de fusion exprimée par ce phage. Les nucléotides sont représentés sur la ligne supérieure et les aminoacides sur la ligne inférieure. Ils sont les uns et les autres numérotés sur la colonne de droite. Les nucléotides aux extrémités, représentés en petites lettres, correspondent aux séquences de la béta-galactosidase.
  Les séquences soulignées se réfèrent aux deux sites analysés dans les figures 9a et 9b.

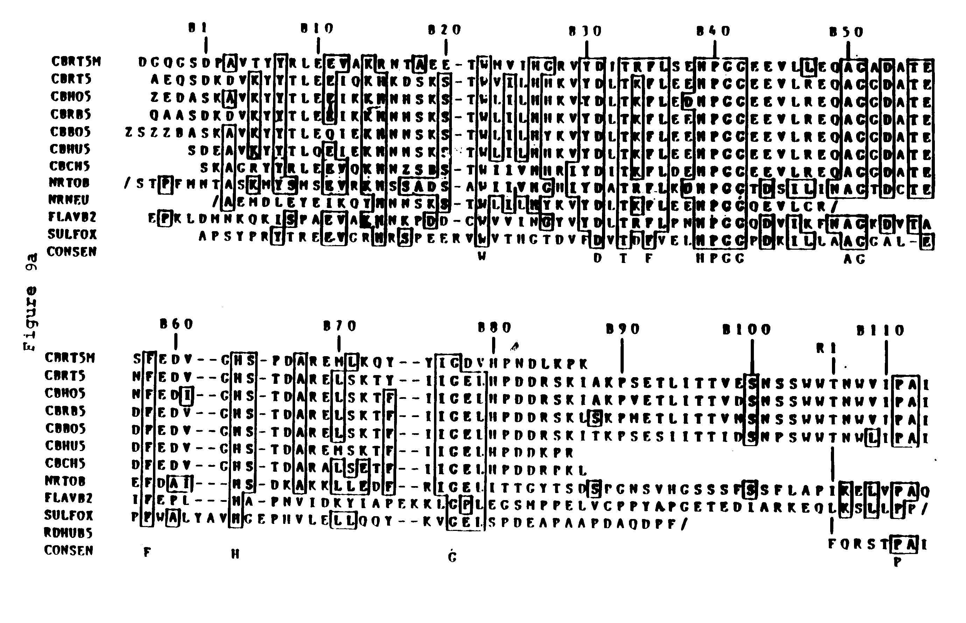

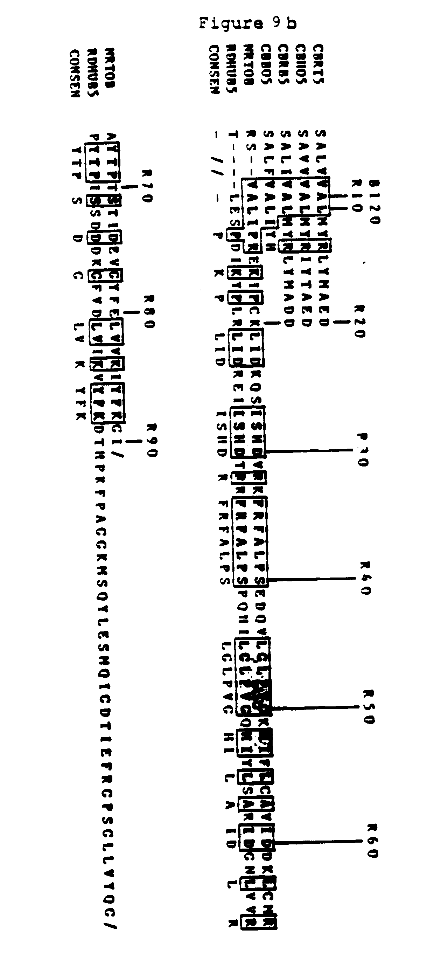

- Les figures 9a et 9b représentent l'alignement de la séquence d'aminoacides de la nitrate réductase dans le site d'attachement à l'haemine et les sites FAD/NADH avec les protéines de la superfamille du cytochrome b5 et la réductase de cytochrome b5 d'erythrocyte humain (RDHUB5). Les séquences identiques avec la NR de tabac sont encadrées. Les restes du site d'attachement à l'haemine ont été numérotés selon les conventions pour le cytochrome b5 de bovin avec les deux histidines liant à l'haemine numérotées His-39 et His-63 (B ?). Le site FAD/NADH a été numéroté à partir du reste N-terminal de la réductase de cytochrome b5.
- La figure 10 représente l'alignement de la séquence d'aminoacide de la nitrate réductase en première ligne et la séquence codante complète correspondante en seconde ligne.

De façon générale, le processus expérimental repose sur le fait que les ARN messagers codant l'apoenzyme de nitrate réductase du tabac peuvent être traduits dans un système acellulaire.

Des fractions d'ARNm polyA$^+$ de la plage 23-32s d'un gradient de saccharose ont été utilisées pour construire une banque d'ADNc dans le vecteur d'expression lambdaGT11 avec une efficacité de clonage d'environ 10$^4$ recombinants/ng d'ARNm. Les clones recombinants ont été triés avec un anticorps polyclonal de lapin dirigé contre la nitrate réductase de maïs qui réagit spécifiquement avec les nitrates réductases de dicotylédones. Parmi 240 000 plages de lyse de recombinants, 8 clones ont été isolés contenant des inserts de tailles s'étalant de 1,6 kb à 2,1 kb et ayant en commun des homologies de séquences. 7 de ces clones contenaient un fragment EcoRI de 1,6 kb interne commun. L'identité de ces clones a été confirmée de la manière suivante : une protéine de fusion de 170 kd pouvant être induite par IPTG et reconnue par l'anticorps de la NR de lapin a été exprimée par un dérivé de lysogène à partir d'un des recombinants. Les anticorps couplant les protéines fusionnées ont été élués et se sont révélés inhibiteurs de l'activité catalytique de la NR de tabac. Les anticorps monoclonaux dirigés contre la NR ont aussi été capables de coupler les protéines hybrides. Quand on a utilisé le fragment EcoRI de 1,6 kb comme sonde pour les expériences de "northern blot", on a détecté un signal correspondant à un ARN de 3,5 kb dans les préparations d'ARNm de N. plumbaginifolia et de tabac mais aucune hybridation croisée avec des ARNm de mais n'a été détectée. La sonde s'est hybridée avec des séquences à faible nombre de copies dans les taches génomiques d'ADN de tabac.

EXEMPLE 1

EXTRACTION ET FRACTIONNEMENT DE L'ARNm PolyA$^+$

L'ARN total a été extrait des tissus de feuilles de tabac selon une procédure proche de Chirgwin (Biochemistry 18, 5294-5299 (1979). Des feuilles de tabac ont été gelées dans N$_2$ liquide et broyées dans un mortier. La poudre a été mélangée rapidement avec 100 ml de tampon Tris-HCl pH 8,0 50 mM contenant de l'EDTA 10 mM, du thiocyanate de guanidinium 5 M, du sodium N laurylsarcosine 2 % et du 2-mercaptoéthanol 5 % (v/v). Après incubation à 65°C pendant 20 minutes et centrifugation à 20 000 g pendant 30 minutes, les surnageants ont été ajustés à 0,1 g/ml de CsCl et étalés sur des coussins de 12 ml

de $C_3Cl$ 5,7 M et EDTA 50 mM, pH 8,0 dans des tubes de 30 ml de polyallomères. Les tubes ont été centrifugés pendant 20 heures à 25 000 rpm dans un rotor SW27 à 15°C. Les culots d'ARN ont été dissous dans du Tris 10 mM pH 8,0 et EDTA 1 mM, et du SDS 0,05 % et mis en incubation à 55°C pendant 30 minutes en présence de 200 $\mu$g/ml de protéinase K. Après le traitement PMSF pour désactiver la protéinase K, des échantillons ont été extraits par le phénol, phénolchloroforme, chloroforme et précipité éthanol. Le rendement était en général de 200 à 300 $\mu$g d'ARN total par gramme de tissu frais. Les ARNm PolyA+ ont été purifiés par deux cycles de sélection d'une colonne d'oligocellulose (PL biochemical) selon la procédure de Bantle (1978) (Anal. Biochem. 70, 75-85) comprenant une étape de dénaturation de l'ARN dans du DMSO 80 % à 55°C pour le second cycle de sélection.

Les ARNm PolyA+ représentaient en général 1 à 1,2 % de l'ARN de feuille total et contenaient moins de 10 % d'ARN ribosomal. On a fractionné les ARNm PolyA+ (100 $\mu$g/tube) sur des gradients de saccharose linéaires de 12 ml de 5 à 20 % faits dans un tampon TE par centrifugation pendant 16 heures à 100 000 g dans un rotor SW40 selon la procédure décrite par Commere dans Plant. Sci. (1986) 44, 191-203.

## EXEMPLE 2

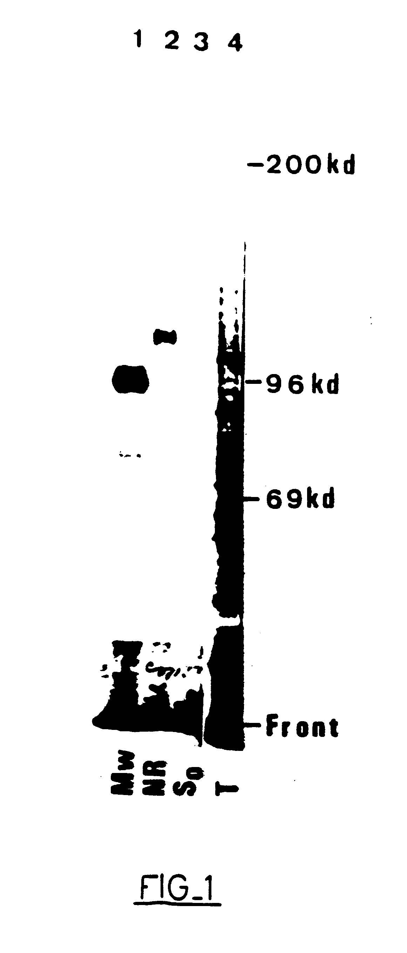

### TRADUCTION ACELLULAIRE D'ARNm ET IMMUNOSELECTION DES PRODUITS DE TRADUCTION (figure 1)

Des ARNm PolyA+ totaux ou fractionnés ont été traduits dans un système de traduction acellulaire de reticulocyte de lapin (Amersham) utilisant de la méthionine marquée $S^{35}$ (900 Ci mmole, 1 mCi/ml, Amersham). On a ajouté environ 0,05 à 0,1 $\mu$g d'ARNm et 1,2 $\mu$Ci de méthionine $S^{35}$ par $\mu$l de lysat. Après incubation pendant 1 heure à 30°C, des prélèvements du mélange de traduction ont été analysés par SDS-PAGE. Les produits de traduction correspondant à 50 $\mu$g de lysat ont été immunosélectionnés pour des fragments de polypeptides de NR en les passant à travers une colonne de protéine A-Sepharose 4B sur laquelle 20 $\mu$l d'antisérum spécifique de NR avaient été fixés dans du tampon TNE (50 mM Tris-HCl pH 7,5, EDTA 1 mM, Tween 20 0,5 % (w/v), 150 mM NaCl, $NaN_3$ 0,2 %). Après adsorption du lysat dilué dans le tampon TNE, la colonne a été lavée avec du tampon TNE contenant de la méthionine froide 1 mM et du NaCl 1 M, ainsi que du tampon Tris-Hcl 50 mM pH 7,5. Le matériau lié à la protéine A a été ensuite élué et dénaturé dans un tampon Laemmli pour SDS-PAGE.

Le criblage immunologique d'une banque d'expression d'ADNc demande que le nouveau produit de traduction puisse être reconnu par les anticorps spécifiques dressés contre des chaînes polypeptidiques développées dans des cellules d'eucaryotes. Ces anticorps peuvent ne pas reconnaître les chaînes d'antigènes non modifiés synthétisés dans l'hôte de clonage E. coli. La démonstration préalable de la faisabilité de cette approche dans le cas de la NR a été obtenue en montrant qu'un anticorps polyclonal contre la NR de maïs (alpha MNR) pouvait être utilisé pour immunosélectionner une chaîne de polypeptides de NR de tabac parmi les produits de traduction acellulaire d'ARN PolyA+ total extrait des feuilles (Figure 1). L'anticorps polyclonal a été dressé contre la NR de feuille de maïs. L'anticorps polyclonal a reconnu spécifiquement la NR de tabac dans les "western blots" et les produits de traduction acellulaire correspondants dans les lysats de réticulocyte de lapin.

Les signaux obtenus étaient très faibles, la NR représentant 2 à 5/10 000 du total des protéines de feuille de tabac solubles. Les produits de traduction acellulaire ont migré près des sous-unités d'enzyme de 110 kd. Des résultats semblables avaient été obtenus antérieurement avec l'ARNm de NR de maïs.

## EXEMPLE 3

### CONSTRUCTION DE BANQUES D'ADNc DE FEUILLES DE TABAC DANS LE PHAGE lambdaGT11

On a synthétisé des ADNc selon Gubler et Hoffmann (1983) (Gene 25, 263-269) avec les modifications suivantes : 200 ng d'ARNm PolyA+ total ont été dénaturés par chauffage à 70°C pendant 2 minutes dans de l'eau et soumis à une réverse-transcription à pH 8,3 en utilisant 14 U de reverse transcriptase AMV (GenoFit) dans un volume final de 40 $\mu$l en présence de 0,1 $\mu$g d'oligo dT (15 mere, Pharmacia) à 42°C pendant 40 minutes. L'efficacité de la synthèse du premier brin variait entre 8 et 25 %. Le second brin a été synthétisé à partir du premier en présence de 0,8 U de RNAse H (BRL), et 4 U de ligase d'ADN de E. coli (Biolabs), utilisant 40 U d'ADN poli holoenzyme (Amersham) dans un volume final de 90 $\mu$l. L'incubation a été effectuée à 12°C pendant 1 heure et ensuite à 16°C pour 1 heure de plus. Les extrémités de l'ADN double brin ont été polies par incubation avec une unité d'ADN polymérase $T_4$ à 37°C

pendant 15 minutes. L'efficacité de la synthèse du deuxième brin était proche de 100 %. L'ADNc obtenu (environ 100 ng) a été ensuite méthylé avec 20 U d'EcoRI méthylase. Pour améliorer l'efficacité du clonage (10 fois) une étape de digestion des régions simple brin a été incluse dans la procédure de synthèse de l'ADNc entre les étapes de polissage et de méthylation. Les ADNc ont été traités par 2 U de nucléase $S_1$ - (Boehringer) dans un volume de 100 $\mu$l pendant 15 minutes à 30°C en vue d'éliminer les extrémités anormales. Ceci a conduit à une baisse de 5 % de la quantité de matière à haut poids moléculaire dans la préparation de l'ADNc. Après addition de "linkers" d'EcoRI à l'ADNc (12 mers phosphorylés, PL, Biochemicals) et deux digestions successives avec EcoRI concentré, les ADNc ont été fractionnés sur une colonne Sephacryl S-300 et la matière correspondant aux ADNc supérieurs à 0,5 kb a été collectée.

L'ADNc a été ligaturé avec le vecteur lambdaGT11 desphosphorylé coupé avec EcoRI dans les conditions spécifiées par Young (1983) (Proc. Nat. Acad. Sci. USA, 80, 1194-1198). Le mélange de ligation a été encapsidé en utilisant les lysats d'encapsidation disponibles dans le commerce (Gigapack, Stratagene Cloning System, Clontech Labs. CA). Les banques ont été examinées quant à leur proportion de recombinants sur souche RY1088 en présence d'Xgal et IPTG. Elles contenaient 70 à 80 % de plaques blanches. Une proportion significative de ces plaques blanches détectées dans les premières banques a tourné au bleu pâle après nouvel examen sur souche RY1090, et manquait d'inserts détectables. On a trouvé que ceci était en relation avec la faible élimination de petits fragments et "linkers" d'ADNc par chromatographie sur colonne Sépharose 4B et était amélioré par l'utilisation de Séphacryl 9300. Après optimisation, les rendements étaient d'environ 50 000 à 100 000 plaques blanches par ng de d'ADNc parmi lesquelles 50 % contenaient des inserts détectables.

Une banque d'ADNc enrichie en séquence d'ARNm de haut poids moléculaire a été réalisée comme suit. On a fractionné 100 $\mu$g d'ARNm sur un gradient de saccharose et on a collecté la région 23-32s (10 $\mu$g). Cette fraction a été ensuite fractionnée dans un gel d'agarose 1,5 % contenant 10 mM d'hydroxyde de méthylmercure. La plage correspondant aux poids moléculaires 2-5 kb a été découpée, les ARNm élués, extraits au phénol et utilisé (100 ng) pour construire une banque d'ADNc.

Le vecteur de clonage lambdaGT11 a été choisi pour construire des banques d'ADNc car on pensait que les fréquences d'ADNc de NR seraient très faibles et qu'il faudrait cribler de grandes banques. Des banques d'ADNc total ont tout d'abord été construites. Les efficacités initiales de clonage et les proportions de recombinants véritables étaient faibles (moins de 10 000 clones/ng d'ADNc) quand on a suivi la procédure de Gubler et Hoffmann (1983) pour la synthèse des ADNc. L'efficacité de clonage a été accrue en incluant un traitement de nucléase $S_1$ doux entre les étapes de "healing" et de méthylation de la synthèse d'ADNc et a permis d'obtenir un rendement de clonage supérieure à $10^5$ recombinants/ng d'ADNc. La qualité des banques d'ADNc a aussi été améliorée en fractionnant les ADNc après addition de "linkers" sur des colonnes Sephacryl S300 à la place de colonnes Sepharose 4B.

Comme l'ARNm codant pour la NR était considéré comme devant être d'une taille supérieure à 3 kb, des préparations d'ARNm ont été fractionnées sur un gradient de saccharose et des molécules avec une constante de sédimentation s'étalant entre 23-32s ont été recueillies. Ces ARNm ont été fractionnés ultérieurement sur un gel d'hydroxyde de méthylmercure et les ARNm migrant dans le domaine de 3 à 4 kb ont été utilisés pour construire une banque d'ADNc (4.$10^5$ clones, 70 % de recombinants). Ces deux étapes de purification représentaient un enrichissement de 5 à 10 fois en ARNm de NR.

EXEMPLE 4

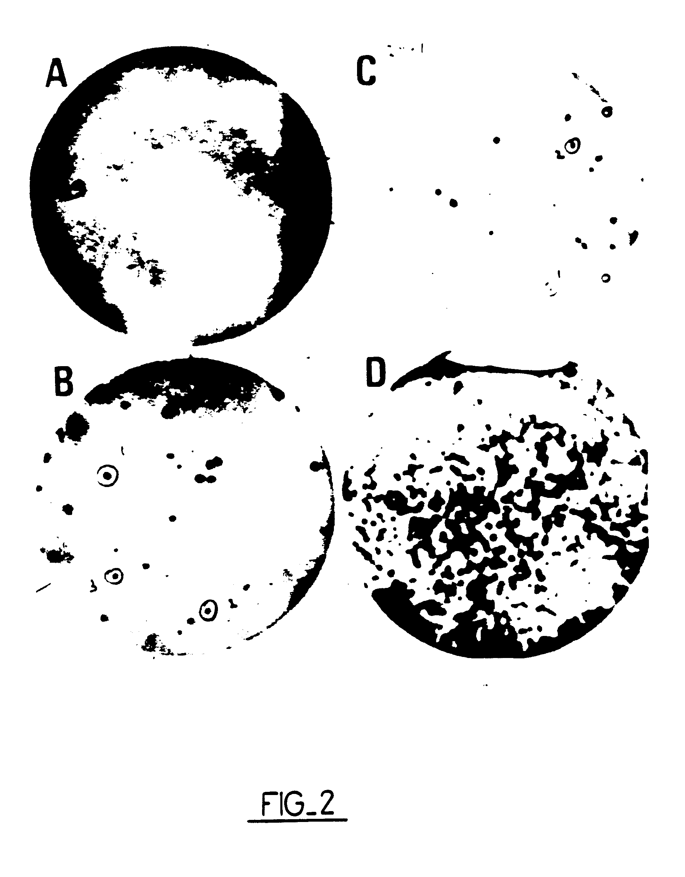

AMPLIFICATION ET CRIBLAGE IMMUNOLOGIQUE DES BANQUES (figure 2)

Les banques ont été amplifiées sur souche RY1088 à une densité de 3.$10^4$ à $10^5$ pFu par boîte de 90 mm. Les plaques ont pu croître à 42°C pendant 4 heures. Il en a résulté une amplification de 1 000 fois. Les banques amplifiées ont été étalées sur souche RY1090 à une densité de 1 à 1,5.$10^4$ pFu par boîte de 90 mm et ont pu se développer à 42°C pendant 3-4 heures. Des filtres de nitrocellulose (Millipore HATF) ont été imprégnés dans de l'IPTG 10 mM puis séchés. Les plages recouvertes de ces filtres ont été mises en incubation pendant la nuit à 37°C puis réfrigérées à 4°C. Les filtres correspondants ont été bloqués avec de la BSA 3 % dans du TBS pendant 30 minutes, mis en incubation avec l'antisérum (dilué 1/200 dans du TBS) pendant 1-2 heures et lavés trois fois dans TBS contenant du Tween 20 à 0,05 %. Les filtres ont été mis en incubation avec du $I^{125}$-protéine A, 0,5 $\mu$Ci dans du TBS contenant 50 g/l de lait écrémé et du Tween 20 à 0,05 % pendant 1-2 heures, lavés trois fois dans du TBS contenant du lait et du Tween 20 puis deux fois dans du TBS. Toutes ces étapes ont été exécutées à température ambiante. Les filtres ont été autoradiographiés sur film Kodak XAR5 avec un écran amplifiant Dupont Cronex pendant 18-24 heures. Une moyenne de 1 à 3 taches ont été détectées sur les filtres. Les parties correspondant à ces taches ont été prélevées avec des pipettes Pasteur par l'extrémité large et mises à incuber toute une nuit à 4°C dans 1 ml de diluant lambda. Environ 3 000 plaques par partie ont été retriées pour confirmation. Deux cycles de triage supplémentaire à basse densité (100-200 plaques/boîte) ont été nécessaires pour purifier les recombinants jusqu'à homogénéité.

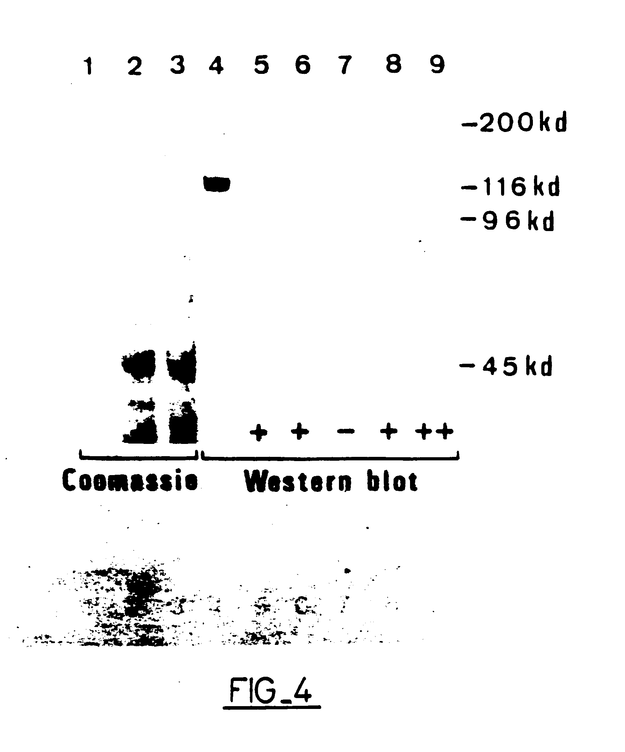

Bien qu'il soit spécifique pour la NR de tabac dans des extraits de feuilles de tabac, l'anticorps alphaMNR a reconnu plusieurs protéines d'E. coli dans des expériences "western blot" (figure 4). Ceci se traduisait par des bruits de fond importants quand les banques étaient filtrées immunologiquement. L'anticorps alphaMNR a donc été mis en incubation avec de grandes quantités d'extraits de protéines bruts d'E. coli et s'est confirmé comme encore capable de détecter de la NR de tabac au niveau du ng. Parmi un total de 40 clones initialement sélectionnés par triage de 240 000 plaques de recombinants, 11 clones exprimant de manière reproductible un signal par retriage ont été ensuite purifiés jusqu'à homogénétité. Ceci a demandé, le cas échéant, pour le clone 13-29 à forte expression, le dépôt, le prélèvement et l'amplification de plaques isolées en l'absence d'une induction IPTG pour empêcher l'apparition de clones sans expression pendant la croissance des plaques (figure 2).

## EXEMPLE 5

## PREPARATION D'ADN DE PHAGE ET SOUS-CLONAGE DES INSERTS

La procédure de production de phages sur milieu LB solide contenant du glucose et unification par centrifugation sur gradient de densité de chlorure de césium est pour l'essentiel celle de Huynh et al. (1983) (DNA cloning a practical approach IRL Press, Oxford). L'ADN a été extrait à partir de $10^{12}$ phages par traitement avec du formamide 50 % dans du Tris HCl 0,2 M pH 8,5, EDTA 20 mM pendant 2 heures à température ambiante. L'ADN a été précipité à l'éthanol, dissous dans un tampon TE contenant 100 $\mu$g/ml de protéinase K et mis à incuber 2 heures à 37°C. Après extraction de l'éthanol, l'ADN a été précipité et utilisé pour une analyse de restriction. Les fragments EcoRI excisés du vecteur ont été sous-clonés dans le site EcoRI desphosphorylé du plasmide pUC9 selon des procédures standards.

## EXEMPLE 6

## REACTIFS IMMUNOLOGIQUES POUR LE TRIAGE DES BANQUES

La purification de la nitrate réductase du mais et l'obtention de l'anticorps polyclonal du lapin (alphaMNR) contre cette enzyme ont déjà été décrites. Pour neutraliser les immunoglobulines anti E. coli contre l'anticorps alphaMNR, le sérum a été dilué 5 fois dans du TBS BSA et mis à incuber avec deux volumes d'un lysat frais d'E. coli (20 mg/ml protéine) préparé par décongélation et sonication de cellules BNN97 en phase de croissance exponentielle et induites pour la production de lambdaGT11, 90 minutes avant récolte. Après incubation pendant 2 heures à 4°C en brassant, le mélange a été centrifugé dans des tubes Eppendorf et la procédure d'addition de lysats et clarification a été répétée deux fois. Le sérum résultant a été maintenu à 4°C avec de l'azide et a pu être utilisé plusieurs fois pour le triage.

## EXEMPLE 7

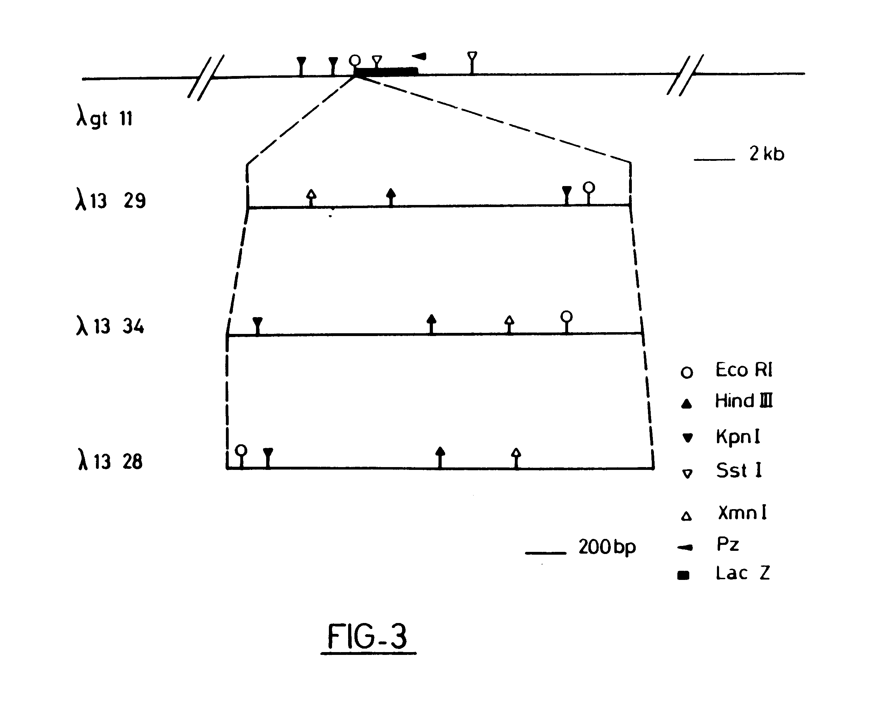

## ANALYSE DES CLONES RECOMBINANTS (figure 3)

Les clones purifiés ont été testés quant à la présence d'inserts d'ADN par digestion d'EcoRI. 7 clones (13-18, 13-20, 13-27, 13-29, 13-33, 13-34, 13-36) contenaient un insert d'EcoRI de 1,6 kb similaire, bien qu'ils donnaient des signaux de différentes intensités par détection immunologique. Le fragment d'EcoRI de 1,6 kb du clone 13-36 a été sous-cloné dans du pUC9 et utilisé comme sonde pour l'étude d'homologies d'inserts parmi les 10 autres recombinants. 7 clones (13-18, 13-20, 13-27, 13-28, 13-29, 13-33, 13-34) se sont hybridés par croisement avec cette sonde. Le clone 13-28 contenait un insert EcoRI de 2 kb. La structure et l'orientation des ADNc dans le site de clonage du vecteur ont été étudiées en utilisant les enzymes de restriction KpnI et SstI. On a trouvé que la taille des inserts dans la majorité des recombinants était supérieure à 1,6 kb donc que le fragment EcoRI de 1,6 kb était interne aux ADNc et entouré de petits fragments d'ADNc qui n'avaient pas été vus sur les digestions d'EcoRI, colorées de bromure d'éthidium, des ADN des phages recombinants. Les inserts ont été trouvés orientés dans les deux directions possibles. Des diagrammes physiques de 3 de ces inserts sont présentés figure 3. De leur comparaison on peut conclure que 2,1 kb d'ARNm ont été clonés. L'absence de certains sites de restriction dans les fragments d'EcoRI de 1,6 kb dans l'insert du clone 13-28 suggère une hétérogénéité parmi les populations d'ARN étudiées. Les 3 clones recombinants restants contenaient des petits inserts hétérologues.

EXEMPLE 8

## EXPRESSION D'UNE PROTEINE DE FUSION DANS LE CLONE RECOMBINANT 13-29 (figure 4)

Des lysogènes de phages 13-29 et 13-36 portant des inserts orientés dans les directions opposées ont été construits dans une souche RY1089. Ces lysogènes ont été testés quant à l'expression d'une protéine hybride par analyse de "western blot" (figure 4). En utilisant l'anticorps polyclonal alphaMNR, une protéine de poids moléculaire d'environ 175 kd a été trouvée dans les extraits d'un lysogène pour phage 13-29 induit avec IPTG, mais cela n'a pas été le cas pour les contrôles non induits. Cette protéine hybride a aussi été détectée par coloration au bleu de coomassie d'extraits fractionnés du SDS-PAGE correspondant (figure 4). Aucune protéine hybride n'a été détectée dans un lysogène pour phage le 13-36. Ces résultats concordent avec l'insert du clone 13-29 qui est intégré en phase avec la séquence codante de la beta-galactosidase et codant pour une chaîne polypeptidique de taille approximative de 60 kd compatible avec la taille de l'insert d'ADNc correspondant. L'isolement par criblage immunologique du clone 13-36 portant un insert orienté à l'opposé du gène de beta-galactosidase, et l'étude du lysogène correspondant, confirment la faible expression des séquences insérées sous le contrôle d'un autre promoteur de phage dans lambdaGT11, comme cela a été discuté par Lapeyre (1985) (Theses Université de Toulouse, Rangueil, France). L'absence de détection de protéines hybrides dans les "western blots" d'extraits d'un lysogène du clone recombinant 13-36 peut être attribué au bas niveau d'expression de cette protéine hybride.

Les lysogènes ont été obtenus par infection de la souche RY1089 HFI par des phages recombinants. Des extraits bactériens ont été obtenus à partir de lysogènes induits ou non par IPTG comme décrit par Riva, 1985, Thèses PhD, Université Paris VI, France). Les extraits ont été analysés par électrophorèse SDS-PAGE, des expériences du "western blot" des protéines fractionnées sur nitrocellulose par électrotransfert, immunodétection avec l'antisérum alphaMNR et un deuxième antisérum de mouton dirige contre les IgG de lapin couplé avec de la peroxydase (Cherel, 1985, Biochem. Biophys. Res. Comm., 129, 686-693).

## EXEMPLE 9

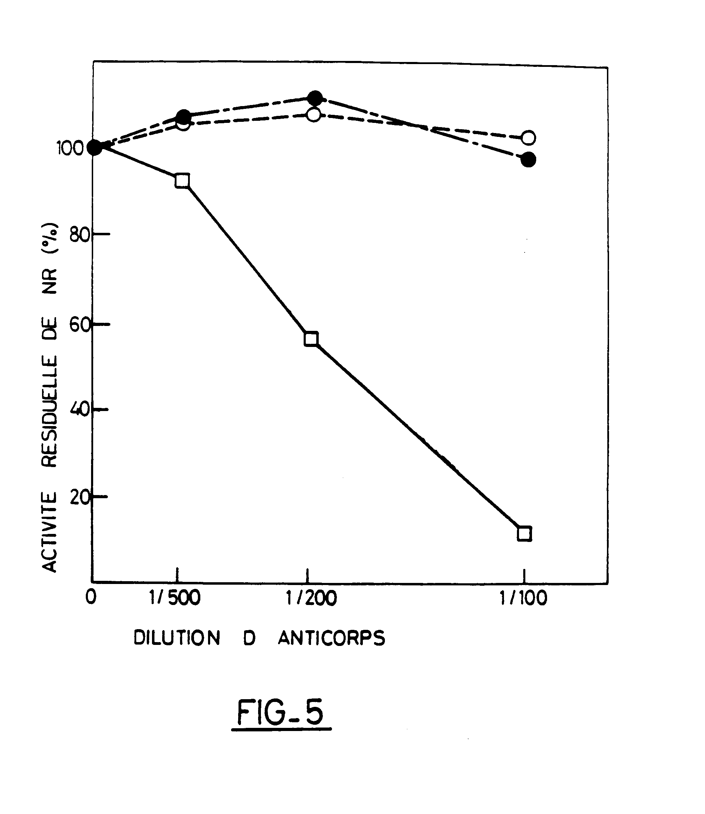

## CONFIRMATION DE L'IDENTITE DES ADNc CLONES PAR IMMUNOSELECTION DES ANTICORPS INHIBANT L'ACTIVITE DE LA NITRATE REDUCTASE DE TABAC (figure 5)

Bien que l'antisérum alphaMNR utilisé pour le criblage ait semblé plutôt spécifique à l'égard de la NR de tabac, l'antisérum peut avoir contenu une sous-classe mineure d'immunoglobuline reconnaissant d'autres protéines de tabac. Ceci pourrait conduire à l'isolement de "faux positifs". Pour tester cette possibilité, des cellules de la souche RY1090 d'E. coli ont été infectées par des phages lambdaGT11 et 13-29. Après induction par IPTG, les protéines synthétisées ont été transférées sur des filtres de nitrocellulose. Les anticorps de contrôle (alpha SO) et polyclonaux (alphaMNR) ont été mis en incubation avec les filtres et les immunoglobulines immunosélectionnées correspondantes ont été éluées des filtres à pH faible. Ces anticorps sélectionnés ont été ensuite analysés quant à leur faculté d'inhiber l'activité réductrice de nitrate de la NR de tabac. Comme on peut le voir figure 5, les protéines exprimées par le clone 13-29 ont pu se lier spécifiquement, dans le sérum alphaMNR, avec les anticorps inhibiteurs de la NR de tabac. Une inhibition à 40 % de l'activité de la NR de tabac a été obtenue avec une dilution
à 1/200 des immunoglobulines sélectionnées, ce qui est raisonnablement en accord avec l'activité inhibitrice du sérum alphaMNR (70 % d'inhibition à dilution 1/200 pour le sérum). Ceci suggère également que la quantité de protéines hybrides disponibles pour l'immunosélection dépassait largement la quantité d'immunoglobulines adsorbée dans le sérum. Il a été trouvé que, certainement, l'antisérum alphaMNR déjà utilisé pour la confirmation immunologique des clones positifs, était inapproprié pour le triage supplémentaire des nouveaux recombinants, probablement à cause d'une diminution de son titre en immunoglobulines dirigées contre la NR.

EXEMPLE 10

RECONNAISSANCE DE LA PROTEINE HYBRIDE EXPRIMEE PAR LE CLONE 13-29 PAR DES ANTICORPS MONOCLONAUX DIRIGES CONTRE LA NITRATE REDUCTASE (tableau 1)

Trois anticorps monoclonaux de NR dressés contre la NR de tabac et maïs (tableau 1) ont été utilisés pour caractériser davantage les protéines hybrides. L'anticorps monoclonal 96(9)25 reconnait la NR naturelle d'une variété de plantes et est inhibiteur de l'activité de l'enzyme. Les anticorps monoclonaux 7-(113)NP15 et 24(48)NP19 reconnaissent la NR naturelle du tabac mais n'ont pas d'incidence sur l'activité de l'enzyme. Pour contrôle, on a utilisé un anticorps monoclonal GET dirigé contre l'enveloppe glycosylée d'un coronavirus de porc. Les résultats sont reproduits au tableau 1. Parmi les quatre anticorps monoclonaux testés, les clones 96(9)25 et 24(48)NP19 ont reconnu spécifiquement les déterminants antigéniques d'un extrait d'un lysogène pour le clone 13-29, fabriqué dans la souche RY1089. Aucune reconnaissance significative n'a été détectée pour les clones 7(113)NP15 et GET. L'anticorps monoclonal 96(9)25 a une affinité supérieure pour la NR de tabac que l'anticorps monoclonal 24(48)NP19. La reconnaissance de la protéine hybride par ces deux anticorps monoclonaux suggère une situation semblable. Au moins deux épitopes différentes peuvent être détectées sur la protéine hybride qui sont ainsi reconnues sur la NR de tabac. L'absence de l'épitope reconnue par l'anticorps monoclonal 7(113)NP15 montre aussi, comme prévu, que la NR de tabac et la protéine hybride sont antigéniquement différentes, à cause de l'absence d'une partie de la séquence codante de la NR dans l'ADNc cloné.

La méthode d'immunosélection des anticorps reconnaissant les protéines hybrides exprimées par les recombinants est la suivante :

Des clones lambdaGT11 du type sauvage et recombinant ont été déposés sur la souche RY1090 dans des boîtes de 90 mm. Après lyse confluente en présence d'IPTG, les protéines des plages ont été transférées sur nitrocellulose. Deux transferts de nitrocellulose ont été saturés pendant 1 heure avec de la BSA 30 mg/ml et après lavage dans du PBS, ils ont été mis à incuber dans les antisérums (5 ml) alphaSo ou alphaMNR dilués à 1/00. Après incubation et lavage des filtres avec PBS, les anticorps adsorbés sur les filtres ont été élués par une incubation de 30 secondes dans 5 ml d'un tampon pH 2,2 de HCl-glycine 0,2 M. Après retrait du filtre, le tampon d'élution a été rapidement neutralisé avec du Tris base et l'antisérum alphaSo non-immun a été ajouté dans un volume final de 10 ml à une dilution de 1/200 fois. Les protéines éluées ont été concentrées et dialysées contre PBS en utilisant une cellule Amicon équipée d'une membrane PM30. Différentes concentrations de ces échantillons ont été ajoutées à une préparation de NR de tabac purifiée. préparée par chromatographie d'affinité. Après 5 minutes d'incubation, l'activité de la NR a été analysée.

La méthode des mesures ELISA d'affinité des anticorps monoclonaux pour les protéines de fusion est la suivante :

L'isolement et la caractérisation de l'anticorps monoclonal 96(9)25 fixant le site actif de NR de différentes plantes ont déjà été décrites par Cherel (1985). Plusieurs anticorps monoclonaux reconnaissant l'enzyme du tabac ont été obtenus en utilisant une méthode semblable. La mesure ELISA d'affinité des anticorps monoclonaux pour les protéines de fusion a été réalisée en utilisant un test à deux sites selon la procédure décrite par Cherel (1986) (Plant. Physiol. 81, 376-378). Les réactifs ont été versés dans les plaques de microtitrage dans l'ordre suivait : anticorps monoclonal du fluide ascitique, extrait d'E. coli de lysogène, antisérum alphaMNR et IgG anti-lapin, conjugué d'alkaline phosphatase.

TABLEAU 1

| MESURE ELISA DE L'AFFINITE DES ANTICORPS MONOCLONAUX DIRIGES CONTRE LA NR, POUR LES PROTEINES HYBRIDES EXPRIMEES PAR LE CLONE 13-29 | | | |
|---|---|---|---|
| Anticorps Monoclonal | Spécificité | Extraits bactériens testés apres induction IPTG | |
| | | RY1089 (lambdaGT11) | RY1089 (13-29 |
| 96(9)25 | NR de tabac et maïs naturelle | 0,06 | 0,457 |
| 7(113)NP15 | NR de tabac naturelle | 0,019 | 0,004 |
| 24(48)NP19 | NR de tabac naturelle | 0,031 | 0,272 |
| GET | Coronavirus de porc | 0,003 | 0,057 |

EXEMPLE 11

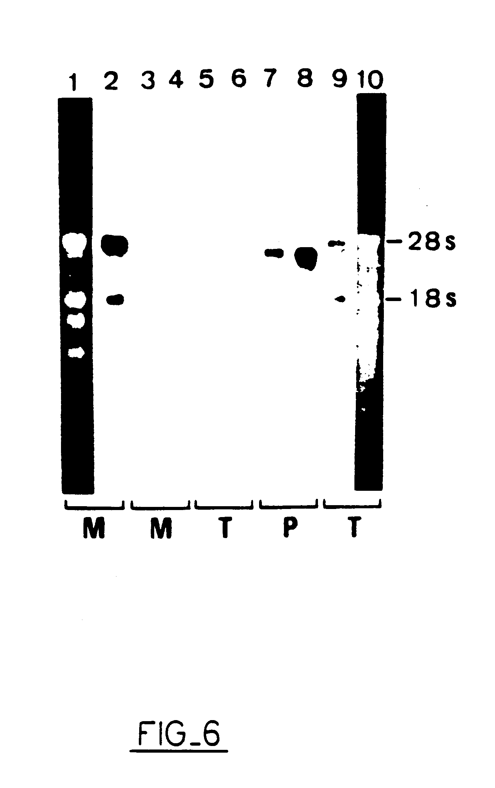

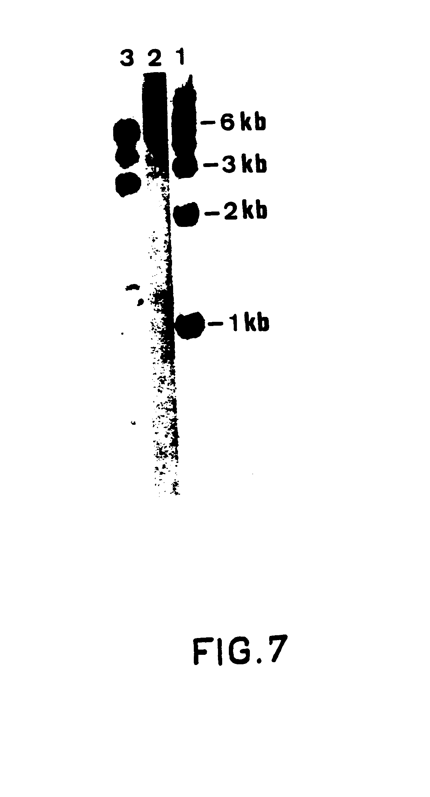

ANALYSES "NORTHERN" et "SOUTHERN BLOT" (Figures 6 et 7)

1) Analyse de "Northern blot" d'ARNm s'hybridant avec le fragment EcoRI de 1,6 kb (figure 6)

Les ARN messagers ont été extraits de feuilles de tabac, de maïs et de N. plumbaginifolia, recueillies sur des plantes cultivées dans un milieu contenant du nitrate. Les "northern blot" des ARNm fractionnés sur des gels de formaldéhyde-agarose ont été sondés avec l'insert d'ADNc de 1,6 kb (figure 6). Un signal d'hybridation a été détecté après 3 jours d'exposition dans des préparations de tabac et N. plumbaginifolia. Aucun signal n'a été détecté, même après exposition prolongée dans le cas des ARNm de maïs. L'hybridation des mêmes taches avec une unité de répétition (motif) d'ADN de blé a montré que les ARNm homologues à l'insert de 1,6 kb avaient migré légèrement plus vite que l'ARN ribosomal de 28 s. Une taille de 3,5-3,6 kb était assignée aux ARNm quand les migrations étaient exécutées dans des gels de méthylmercurhydroxyde dans des conditions totalement dénaturées en présence de marqueurs de poids moléculaire d'ADN monobrin.

2) Analyse de "southern blot" des séquences génomiques homologues au fragment EcoRI de 1,6 kb (figure 7)

La complexité génomique des séquences homologues du fragment d'EcoRI de 1,6 kb a été étudiée en utilisant l'ADN extrait du tabac. Les résultats de "southern blot" effectuée dans des conditions stringentes sont présentés figure 7. Les signaux correspondant aux petits nombres de copies de séquences correspondantes ont été détectés dans les digestions comme le suggérait la comparaison avec les signaux d'hybridation obtenus en sondant avec un motif d'ADNr de blé. Quand l'ADN génomique était coupé par EcoRI, un signal était détecté dans les positions d'un fragment de 3,4 kb et un de 5 kb, et une hybridation faible était aussi observée correspondant à un fragment de 2,9 kb. Deux gènes de structure fonctionnels sont censés être présents dans le génome du tabac. Le fait que le fragment EcoRI de 1,6 kb soit interne à l'ADNc suggère que des séquences intervenant doivent être présentes dans la zone correspondante d'au moins un des deux gènes de structure.

3) Méthode d'expérimentation "northern et southern blot"

Les ARNm ont été fractionnés par électrophorèse dans des gels d'agarose 1,5 % d'hydroxyde de méthylmercure 10 mM pour le dosage précis des poids moléculaires ou dans des gels d'agarose 1,5 % formaldéhyde 3,0 % pour les expériences banales. Après fractionnement les ARNm ont été transférés sur des membranes Hybond C (Amersham) selon Thomas (1980) (Proc. Natl. Acad. Sci. USA, 77, 5201-5205).

L'ADN total a été extrait de jeunes feuilles de tabac selon le mode opératoire décrit par Dellaporta (1983) (Plant. Mol. Biol. Reporter, 1, 19-21), purifié par centrifugation d'équilibre de gradient de CsCl, et

utilisé pour l'analyse "southern blot" conformément à Deshayes (Embo. J. 4, 2731-2737 (1985).

Les "northern et southern blots" ont été identifiés à l'aide de sondes comportant le fragment EcoRI de 1,6 kb d'un phage 13-29 purifié par centrifugation de gradient de saccharose ou avec le pTA71 marqué au phosphore 32 portant un insert d'ADNr de blé de 9 kb. L'hybridation a été effectuée dans du formamide 50 % à 45°C et lavé dans des conditions stringentes (3 lavages à température ambiante dans SDS 0,1 %, 2xSSC, suivis par 2 lavages de 30 minutes à 65°C dans SDS 0,1 %, 0,2xSSC, selon Maniatis (1982) (Molecular Cloning: A laboratory manual, Cold Spring Harbor Laboratory).

EXEMPLE 12

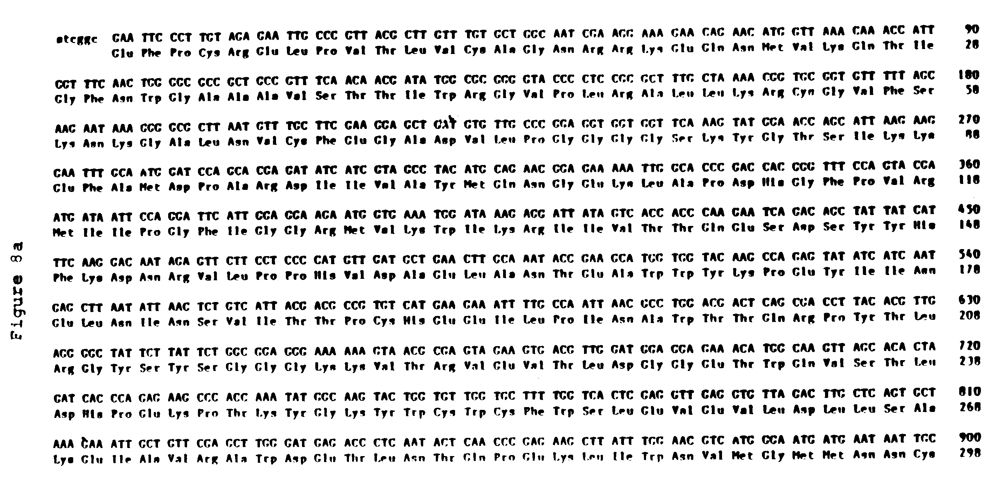

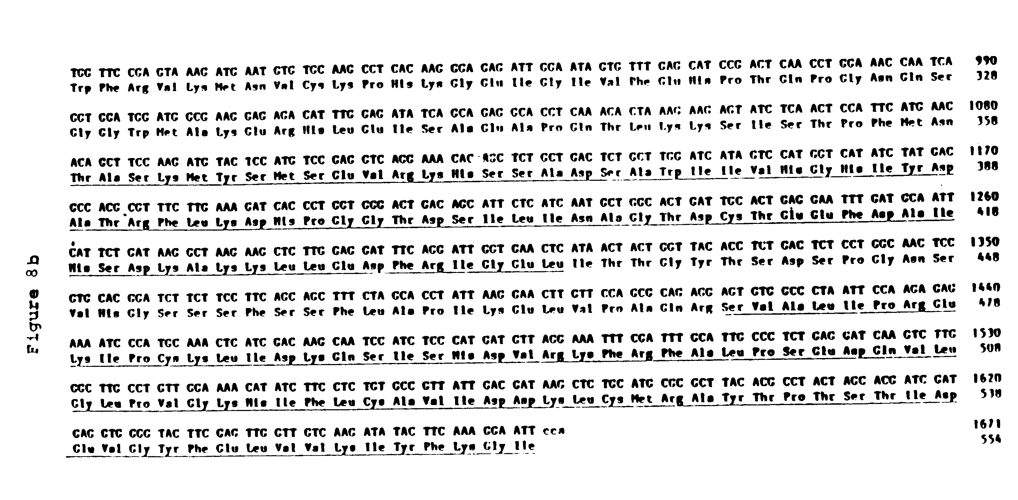

SEQUENCE CODANTE PARTIELLE DES FIGURES 8 ET 9

La séquence nucléotidique des figures 8a et 8b provient du clone 13-29. Longue de 1662 bases, elle comprend les sites EcoRI, mais ne contient pas de séquences polyadénylées.

Parmi les six cadres de lecture possibles de la séquence, un seul se fait sans interruption par un cordon stop et code pour une chaîne polypeptidique de 554 aminoacides avec une masse moléculaire de 62,175 dalton. Ce cadre de lecture ouvert correspond à une fusion de traduction avec la séquence de la bétagalactosidase du vecteur. Ce cadre de lecture représente environ 55 % de la taille de l'apoenzyme de nitrate réductase de tabac.

Comme présentés sur les figures 8 (a-b) et 9 (a-b), deux domaines d'homologie importante sont observés.

De la position 360 à 435, la nitrate réductase de tabac présente une homologie de séquence de 35 à 50 % avec les protéines de la superfamille du cytochrome b5, soit avec le cytochrome b5, le flavocytochrome b2 ou la sulfite oxydase selon Guiard et Lederer (1979a) (31). Le et Lederer (1983) (29) ont isolé par protéolyse à la chymotrypsine, le site d'attachement à l'haemine de la nitrate réductase de Neurospora crassa et montré son homologie avec la famille du cytochrome b5.

Une homologie entre la séquence d'aminoacide de la nitrate réductase de tabac et le domaine d'attachement à l'haemine de la nitrate réductase fongique est également observée comme on s'y attendait.

De la position 476 à 552, la séquence d'aminoacides de la nitrate réductase de tabac présentait une identité de 57 % avec la séquence N-terminale de la réductase de cytochrome b5 humaine (Yubisui et al. 1984) (27). Cette flavoenzyme FAD est connue pour former un complexe binaire avec le cytochrome b5 en effectuant un transfert d'électrons à Partir du NADH pour divers processus physiologiques d'oxydoréduction.

Comme l'ont montré Le et Lederer 1983 (29), le site d'attachement à l'haemine de la nitrate réductase de Neurospora crassa est un membre de la superfamille du cytochrome b5. Nos résultats montrent que l'enzyme de tabac présente également une homologie significative avec différents membres de la superfamille. L'homologie du cytochrome b5 recouvre entièrement le domaine d'attachement à l'haemine des diverses protéines. Cette séquence définit donc vraisemblablement le site d'attachement à l'haemine de nitrate réductase de tabac.

Les treize aminoacides conservés de la superfamille (Lederer et al. 1985) (30) sont présents dans notre séquence de nitrate réductase de tabac qui apparaît plus proche du cytochrome b5 de poulet que des autres membres de la superfamille (figures 9 a-b).

La relative différence existant entre les nitrates réductases de plantes et de champignons tend à indiquer une évolution dissemblable pour cette enzyme.

Une homologie significative de la séquence codante de l'ADNc cloné avec la réductase cytochrome b5 flavoprotéine humaine a aussi été détectée. Cette protéine est connue pour catalyser la réduction du cytochrome b5 utilisant NADH comme donneur d'électrons et FAD comme intermédiaire d'oxydoréduction. Ceci peut être comparé fonctionnellement à la réaction catalytique de réduction des nitrates mettant en oeuvre NADH, FAD et le cytochrome b5 comme cofacteurs. Ceci suggère que cette seconde séquence homologue est le fragment N-terminal du site NADH/FAD de nitrate réductase de tabac.

EXEMPLE 13

SEQUENCE CODANTE COMPLETE DE LA NITRATE REDUCTASE DE TABAC

La séquence de nucléotides de la nitrate réductase de tabac a été obtenue comme suit.

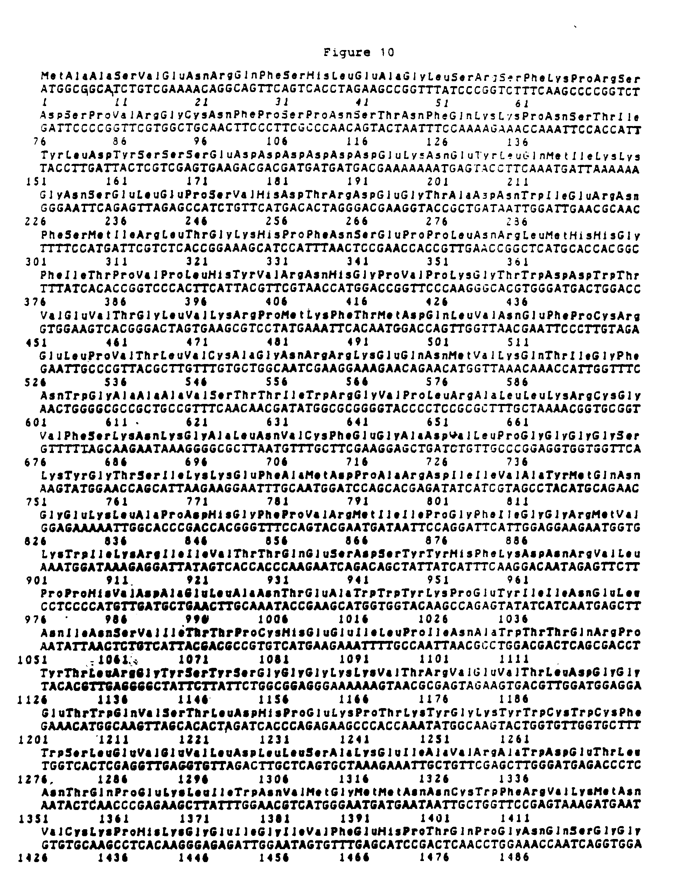

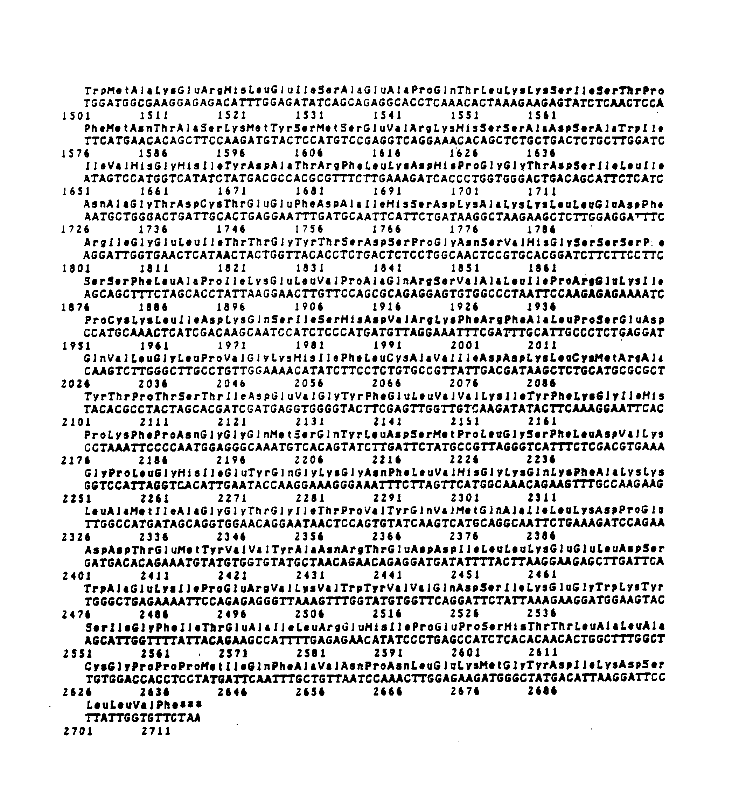

L'insert d'ADNc du phage recombinant λ 13-29, dont la séquence a été décrite ci-dessus, a été utilisé pour cribler des librairies génomiques de tabac faites dans les phages EMBL 3B et EMBL 4. Un phage 3.12 portant les séquences homologues de l'ADNc a ensuite été caractérisé. L'insert de ce phage a été sous-cloné dans pUC 9 et des fragments clonés ont été utilisés pour des expériences d'hybridation de northem blot. L'ADNc partiel a été purifié à partir de la librairie d'ADNc en utilisant des fragments génomiques s'hybridant avec de l'ARNm de nitrate réductase comme sondes. Les séquences des fragments génomiques et les clones d'ADNc ont été réalisées en double brins en utilisant la méthode au didéoxy de Sanger. Les séquences de nucléotides et les séquences d'aminoacides de la nitrate réductase de tabac, qui apparaissent à la figure 10, ont été déduites à partir de l'analyse des données de séquences et numérotées à patir du codon start ATG.

REFERENCES BIBLIOGRAPHIQUES

(1) BANTLE J.A., MAXWELL I.H. et HAHN W.E. (1978) Anal. Biochem. 70, 75-85.

(2) CAMPBELL W.H. et SMARELLI J. (1983) Phytochem 225, 2375-2382.

(3) CHEREL I., GROSCLAUDE J. et ROUZE P. (1985) Biochem. Biophys. Res. Comm., 129, 686-693.

(4) CHEREL I., MARION-POLL. A., MEYER C. et ROUZE P. (1986) Plant Physiol., 81, 376-378.

(5) CHIRGWIN J.M., PRZYBYLA A.E., MacDONALD R.J. et RUTTER W.J. (1979) Biochemistry 18, 5294-5299.

(6) COMMERE B., CHEREL I., KRONENBERGER J., GALANGAU F. et CABOCHE M. (1986), Plant Sci., 44, 191-203.

(7) DELLAPORTA S.L., WOOD J. et HICKS J.B. (1983) Plant Mol. Biol. Reporter, 1, 19-21.

(8) DESHAYES A., HERRERA ESTRELLA L. et CABOCHE M. (1985) EMBO J., 4, 2731-2737.

(9) GERLACH W.L. et BEDBROOK J. (1979) Nucleic Acid Res., 7, 1869-1885.

(10) GUBLER U. et HOFFMANN B.J. (1983) Gene, 25, 263-269.

(11) HOWARD W.D. et SOLOMONSON L.P. (1982) J. Biol. Chem. 257, 10243-10250.

(12) HEWITT E.J. (1975) Annu. Rev. Plant Physiol., 26, 73-100.

(13) HUYNH T.V., YOUNG R.A. et DAVIS R.W. (1984) in Glover, D. (Eds) DNACloning techniques: A practical approach. IRL Press, Oxford.

(14) KLEINHOFS A., WARNER R.L. et NARAYANAN K.R. (1985) Oxford Surveys of Plant Molecular and Cell Bioiogy, 2, 91-121.

(15) LAPEYRE B. (1985) PhD Thesis Université de Toulouse. Rangueil. France.

(16) MANIATIS T., FRITSCH E.F. et SAMBROOK J. (1982) Molecular cloning: A laboratory manual, Cold Spring Harbor Laboratory.

(17) MILLER J.H. (1972) Experiments in Molecular Genetics, Cold Spring Harbor Laboratory, New York.

(18) RASUL CHAUHRY G. et MacGREGOR C.H. (1983) J. Bacteriol., 154, 387-394.

(19) REDINBAUGH M.G. et CAMPBELL W.H. (1985) J. Biol. Chem., 260, 3380-3385.

(20) REDINBAUGH M.G. et CAMPBELL W.H. (1983) Biochem. Biophys. Res. Comm., 114, 1182-1188.

(21) RIVA M. (1985) PhD Thesis, Université Paris VI, France.

(22) THOMAS S.P. (1980) Proc. Natl. Acad. Sci. USA, 77, 5201-5205.

(23) VIEIRA J. et MESSING J. (1982) Gene, 19, 259-268.

(24) YOUNG R.A. et DAVIS R.W. (1983) Proc. Nat. Acad. Sci. USA, 80, 1194-1198.

(25) LEDERER F., GHRIR R., GUIARD B., CORTIALS S., ITO A. (1983) Two homologous cytochromes b5 in a single cell. Eur. J. Biochem. 132 : 95-102

(26) OZOLS J., HEINEMANN F.S. (1982) Chemical structure of rat liver cytochrome b5. Isolation of peptides by high-pressure liquid chromatography. Biochem. Biophys. Acta 704 : 163-173

(27) OZOLS J., GERARD C., NOBREGA F. (1976) Proteolytic cleavage of horse liver cytochrome b5. Primary structure of the heme-containing moiety. J. Biol. Chem. 251 : 6767-6774

(28) NOBREGA F.G., OZOLS J. (1971) Amino acid sequences of tryptic peptides of cytochromes b5 from microsomes of human,monkey, procine and chicken liver. J; Biol. Chem. 246 : 1706-1717

(29) LÊ KHD, LEDERER F., (1983) On the presence of a hemebinding domain homologous to cytochrome b5 in Neurospora crassa assimilatory nitrate reductase. EMBO J.2 : 1909-1914

(30) LEDERER F., CORTIALS S., BECAM A-M., HAUMONT P.Y., FEREZ L. (1985) Complete amino acid sequence of flavocytochrome b2 from baker's yeast. Eur. J. Biochem. 152 : 419-423
(31) GUIARD B., LEDERER F. (1979) Amino acid sequence of the b5-like heme-binding domain from chicken sulfite oxidase. Eur. J. Biochcm. 100 : 441-453

**Revendications**
**Revendications pour les Etats contractants suivants : AT, BE, CH, LI, DE, FR, GB, GR, IT, LU, NL, SE**

1. Vecteur de clonage comportant un ADN complémentaire de l'ARNm de la nitrate réductase de tabac dans lequel l'insert d'ADNc est d'au moins 1,6 kb et code pour plus de 50% de l'ARNm de la nitrate réductase de tabac, comportant la séquence codante de nucléotides suivante :

```
CGA GCT TGG GAT GAG ACC CTC AAT ACT CAA CCC GAG AAG CTT ATT TGG AAC GTC ATG GGA ATG
ATG AAT AAT TGC TGG TTC CGA GTA AAG ATG AAT GTG TGC AAG CCT CAC AAG GGA GAG ATT GGA
ATA GTG TTT GAG CAT CCG ACT CAA CCT GGA AAC CAA TCA GGT GGA TGG ATG GCG AAG GAG AGA
CAT TTG GAG ATA TCA GCA GAG GCA CCT CAA ACA CTA AAG AAG AGT ATC TCA ACT CCA TTC ATG
AAC ACA GCT TCC AAG ATG TAC TCC ATG TCC GAG GTC AGG AAA CAC AGC TCT GCT GAC TCT GCT
TGG ATC ATA GTC CAT GGT CAT ATC TAT GAC CCC ACG CGT TTC TTG AAA GAT CAC CCT GGT GGG
ACT GAC AGC ATT CTC ATC AAT GCT GGC ACT GAT TGC ACT CAG GAA TTT GAT GCA ATT CAT TCT
CAT AAG GCT AAG AAG CTC TTG GAG GAT TTC AGG ATT GGT GAA CTC ATA ACT ACT GGT TAC ACC
TCT GAC TCT CCT GGC AAC TCC GTG CAC GGA TCT TCT TCC TTC AGC AGC TTT CTA GCA CCT ATT
AAG GAA CTT GTT CCA GCG CAG AGG AGT GTG GCC CTA ATT CCA AGA GAG AAA ATC CCA TGC AAA
CTC ATC GAC AAG CAA TCC ATC TCC CAT GAT GTT AGG AAA TTT CGA TTT GCA TTG CCC TCT GAG
GAT CAA GTC TTG GGC TTG CCT GTT GGA AAA CAT ATC TTC CTC TGT GCC GTT ATT GAC GAT AAG
CTC TGC ATG CGC GCT TAC ACG CCT ACT AGC ACG ATC GAT GAG GTG GCG TAC TTC GAG TTG GTT
GTC AAG ATA TAC TTC AAA GGA ATT

GAA TTC CCT TGT ACA GAA TTG CCC GTT ACG CTT GTT TGT GCT GGC AAT CGA ACG AAA CAA CAG
AAC ATG GTT AAA CAA ACC ATT GGT TTC AAC TGG GGC GCC GCT GCC GTT TCA ACA ACG ATA TGG
CGC GGG GTA CCC CTC CGC GCT TTG CTA AAA CGG TGC GGT GTT TTT AGC AAG AAT AAA GCG GCG
CTT AAT GTT TGC TTC GAA GGA GCT GAT GTG TTG CCC GGA GGT GGT GGT TCA AAG TAT GGA ACC
AGC ATT AAG AAG GAA TTT GCA ATG GAT CCA GCA GGA GAT ATC ATC GTA GCC TAC ATG CAG AAC
GGA GAA AAA TTG GCA CCC GAC CAC GGG TTT CCA GTA CGA ATG ATA ATT CCA GGA TTC ATT GGA
GGA AGA ATG GTG AAA TGG ATA AAG AGG ATT ATA GTC ACC ACC CAA GAA TCA GAC AGC TAT TAT
CAT TTC AAG GAC AAT AGA GTT CTT CCT CCC CAT GTT GAT GCT GAA CTT GCA AAT ACC GAA GCA
TGG TGG TAC AAG CCA GAG TAT ATC ATC AAT GAG CTT AAT ATT AAC TCT GTC ATT ACG ACG CCG
TGT CAT GAA GAA ATT TTG CCA ATT AAC GCC TGG ACG ACT CAG CCA CCT TAC ACG TTG ACG GGC
TAT TCT TAT TCT GGC GGA GGG AAA AAA GTA ACG CGA GTA GAA GTG ACG TTG GAT GGA GGA GAA
ACA TGG CAA GTT AGC ACA CTA GAT CAC CCA GAG AAG CCC ACC AAA TAT GGC AAG TAC TGG TGT
TGG TGC TTT TGG TCA CTC GAG GTT GAG GTG TTA GAC TTG CTC AGT GCT AAA GAA ATT GCT GTT
```

2. Vecteur de clonage selon la revendication 1, caractérisé en ce qu'il comprend la séquence codante de nucléotides complète suivante :

```
                          ATG GCG GCA TCT GTC GAA AAC AGG CAG
TTC AGT CAC CTA GAA GCC GGT TTA TCC CGG TCT TTC AAG CCC
CGG TCT GAT TCC CCG GTT CGT GGC TGC AAC TTC CCT TCG CCC

AAC AGT ACT AAT TTC CAA AAG AAA CCA AAT TCC ACC ATT TAC
CTT GAT TAC TCG TCG AGT GAA GAC GAC GAT GAT GAT GAC GAA
AAA AAT GAG TAC CTT CAA ATG ATT AAA AAA GGG AAT TCA GAG
TTA GAG CCA TCT GTT CAT GAC ACT AGG GAC GAA GGT ACC GCT
GAT AAT TGG ATT GAA CGC AAC TTT TCC ATG ATT CGT CTC ACC
GGA AAG CAT CCA TTT AAC TCC GAA CCA CCG TTG AAC CGG CTC
ATG CAC CAC GGC TTT ATC ACA CCG GTC CCA CTT CAT TAC GTT
CGT AAC CAT GGA CCG GTT CCC AAG GGC ACG TGG GAT GAC TGG
ACC GTG GAA GTC ACG GGA CTA GTG AAG CGT CCT ATG AAA TTC
ACA ATG GAC CAG TTG GTT AAC GAA TTC CCT TGT AGA GAA TTG
CCC GTT ACG CTT GTT TGT GCT GGC AAT CGA AGG AAA GAA CAG
AAC ATG GTT AAA CAA ACC ATT GGT TTC AAC TGG GGC GCC GCT
GCC GTT TCA ACA ACG ATA TGG CGC GGG GTA CCC CTC CGC GCT
TTG CTA AAA CGG TGC GGT GTT TTT AGC AAG AAT AAA GGG GCG
CTT AAT GTT TGC TTC GAA GGA GCT GAT GTG TTG CCC GGA GGT
GGT GGT TCA AAG TAT GGA ACC AGC ATT AAG AAG GAA TTT GCA
ATG GAT CCA GCA CGA GAT ATC ATC GTA GCC TAC ATG CAG AAC
GGA GAA AAA TTG GCA CCC GAC CAC GGG TTT CCA GTA CGA ATG
ATA ATT CCA GGA TTC ATT GGA GGA AGA ATG GTG AAA TGG ATA
AAG AGG ATT ATA GTC ACC ACC CAA GAA TCA GAC AGC TAT TAT
CAT TTC AAG GAC AAT AGA GTT CTT CCT CCC CAT GTT GAT GCT
GAA CTT GCA AAT ACC GAA GCA TGG TGG TAC AAG CCA GAG TAT
ATC ATC AAT GAG CTT AAT ATT AAC TCT GTC ATT ACG ACG CCG
TGT CAT GAA GAA ATT TTG CCA ATT AAC GCC TGG ACG ACT CAG
CGA CCT TAC ACG TTG AGG GGC TAT TCT TAT TCT GGC GGA GGG
AAA AAA GTA ACG CGA GTA GAA GTG ACG TTG GAT GGA GGA GAA
ACA TGG CAA GTT AGC ACA CTA GAT CAC CCA GAG AAG CCC ACC
AAA TAT GGC AAG TAC TGG TGT TGG TGC TTT TGG TCA CTC GAG
GTT GAG GTG TTA GAC TTG CTC AGT GCT AAA GAA ATT GCT GTT
CGA GCT TGG GAT GAG ACC CTC AAT ACT CAA CCC GAG AAG CTT
ATT TGG AAC GTC ATG GGA ATG ATG AAT AAT TGC TGG TTC CGA
GTA AAG ATG AAT GTG TGC AAG CCT CAC AAG GGA GAG ATT GGA
ATA GTG TTT GAG CAT CCG ACT CAA CCT GGA AAC CAA TCA GGT
```

```
GGA TGG ATG GCG AAG GAG AGA CAT TTG GAG ATA TCA GCA GAG

GCA CCT CAA ACA CTA AAG AAG AGT ATC TCA ACT CCA TTC ATG

AAC ACA GCT TCC AAG ATG TAC TCC ATG TCC GAG GTC AGG AAA

CAC AGC TCT GCT GAC TCT GCT TGG ATC ATA GTC CAT GGT CAT

ATC TAT GAC GCC ACG CGT TTC TTG AAA GAT CAC CCT GGT GGG

ACT GAC AGC ATT CTC ATC AAT GCT GGC ACT GAT TGC ACT GAG

GAA TTT GAT GCA ATT CAT TCT GAT AAG GCT AAG AAG CTC TTG

GAG GAT TTC AGG ATT GGT GAA CTC ATA ACT ACT GGT TAC ACC

TCT GAC TCT CCT GGC AAC TCC GTG CAC GGA TCT TCT TCC TTC

AGC AGC TTT CTA GCA CCT ATT AAG GAA CTT GTT CCA GCG CAG

AGG AGT GTG GCC CTA ATT CCA AGA GAG AAA ATC CCA TGC AAA

CTC ATC GAC AAG CAA TCC ATC TCC CAT GAT GTT AGG AAA TTT

CGA TTT GCA TTG CCC TCT GAG GAT CAA GTC TTG GGC TTG CCT

GTT GGA AAA CAT ATC TTC CTC TGT GCC GTT ATT GAC GAT AAG

CTC TGC ATG CGC GCT TAC ACG CCT ACT AGC ACG ATC GAT GAG

GTG GGG TAC TTC GAG TTG GTT GTC AAG ATA TAC TTC AAA GGA

ATT CAC CCT AAA TTC CCC AAT GGA GGG CAA ATG TCA CAG TAT

CTT GAT TCT ATG CCG TTA GGG TCA TTT CTC GAC GTG AAA GGT

CCA TTA GGT CAC ATT GAA TAC CAA GGA AAG GGA AAT TTC TTA

GTT CAT GGC AAA CAG AAG TTT GCC AAG AAG TTG GCC ATG ATA

GCA GGT GGA ACA GGA ATA ACT CCA GTG TAT CAA GTC ATG CAG

GCA ATT CTG AAA GAT CCA GAA GAT GAC ACA GAA ATG TAT GTG

GTG TAT GCT AAC AGA ACA GAG GAT GAT ATT TTA CTT AAG GAA

GAG CTT GAT TCA TGG GCT GAG AAA ATT CCA GAG AGG GTT AAA

GTT TGG TAT GTG GTT CAG GAT TCT ATT AAA GAA GGA TGG AAG

TAC AGC ATT GGT TTT ATT ACA GAA GCC ATT TTG AGA GAA CAT

ATC CCT GAG CCA TCT CAC ACA ACA CTG GCT TTG GCT TGT GGA

CCA CCT CCT ATG ATT CAA TTT GCT GTT AAT CCA AAC TTG GAG

AAG ATG GGC TAT GAC ATT AAG GAT TCC TTA TTG GTG TTC TAA
```

**Revendications pour l'Etat contractant suivant : ES**

1.  Procédé de préparation d'un vecteur de clonage comportant un ADN complémentaire de l'ARNm de la nitrate réductase de tabac, caractérisé en ce qu'on insère un ADNc d'au moins 1,6 kb qui code pour plus de 50% de l'ARNm de la nitrate réductase de tabac, comportant la séquence codante de nucléotides suivante :

```
CCA GCT TGG CAT GAG ACC CTC AAT ACT CAA CCC GAG AAG CTT ATT TGG AAC CTC ATC CGA ATG
ATG AAT AAT TGC TGG TTC CGA GTA AAG ATG AAT GTG TGC AAG CCT CAC AAG CGA CAG ATT CCA
ATA GTG TTT CAG CAT CCG ACT CAA CCT CGA AAC CAA TCA CGT CGA TCG ATG GCG AAG CAG ACA
CAT TTG CAG ATA TCA GCA GAG GCA CCT CAA ACA CTA AAG AAG AGT ATC TCA ACT CCA TTC ATG
AAC ACA GCT TCC AAG ATG TAC TCC ATG TCC GAG GTC ACG AAA CAC AGC TCT GCT GAC TCT GCT
TGG ATC ATA GTC CAT GGT CAT ATC TAT GAC GCC ACG CGT TTC TTC AAA GAT CAC CCT GGT GCG
ACT GAC AGC ATT CTC ATC AAT GCT GGC ACT GAT TGC ACT CAG GAA TTT GAT GCA ATT CAT TCT
CAT AAG GCT AAG AAG CTC TTG GAG GAT TTC ACG ATT GGT GAA CTC ATA ACT ACT GGT TAC ACC
TCT GAC TCT CCT GGC AAC TGG GTG CAC CGA TCT TCT TCC TTC ACC AGC TTT CTA GCA CCT ATT
AAG GAA CTT GTT CCA GCG CAG ACG AGT GTG GCC CTA ATT CCA AGA GAU AAA ATC CCA TCC AAA
CTC ATC GAC AAG CAA TCC ATC TCC CAT GAT GTT AGG AAA TTT CGA TTT GCA TTG CCC TCT GAG
GAT CAA GTC TTG GCC TTC CCT GTT GGA AAA CAT ATC TTC CTC TGT GCC GTT ATT CAC GAT AAG
CTC TGG ATG CGC GCT TAC AGG GCT ACT AGC ACG ATC GAT GAG GTG GCG TAC TTC GAG TTG GTT
GTC AAG ATA TAC TTC AAA GGA ATT
```

```
CAA TTC CCT TCT AGA GAA TTG CCC GTT ACG CTT GTT TGT GCT GGC AAT CGA ACG AAA GAA CAG
AAC ATG GTT AAA CAA ACC ATT CGT TTC AAC TCG CGC CCC GCT CCC CTT TCA ACA ACG ATA TCG
CGC GCG GTA CCC CTC CGC GCT TTG CTA AAA CGG TCC GGT GTT TTT AGC AAG AAT AAA CGG CCG
CTT AAT GTT TGC TTC GAA CGA GCT CAT GTG TTG CCC GGA GGT GGT GGT TCA AAG TAT CGA ACC
AGC ATT AAG AAG CAA TTT GCA ATG GAT CCA GCA CGA GAT ATC ATC GTA GCC TAC ATG CAG AAC
CGA GAA AAA TTG GCA CCC GAC CAC GCG TTT CCA GTA CGA ATG ATA ATT CCA GGA TTC ATT CGA
GGA AGA ATG GTG AAA TCG ATA AAG ACG ATT ATA GTC ACC ACC CAA GAA TCA CAC AGC TAT TAT
CAT TTC AAG GAC AAT AGA GTT CTT CCT CCC CAT GTT GAT GCT GAA CTT GCA AAT ACC GAA GCA
TCG TCG TAC AAG CCA GAG TAT ATC ATC AAT GAG CTT AAT ATT AAC TCT GTC ATT ACG ACG CCG
TGT CAT GAA GAA ATT TTG CCA ATT AAC GCC TCG ACG ACT CAG CUA CCT TAC ACG TTG ACG GGC
TAT TCT TAT TCT GGC GGA GCG AAA AAA GTA ACG CGA GTA GAA GTC ACG TTG GAT GGA GGA GAA
ACA TCG CAA GTT AGC ACA CTA GAT CAC CCA GAG AAG CCC ACC AAA TAT GGC AAG TAC TCG TCT
TGG TCC TTT GGG TCA CTC GAG GTT GAG GTG TTA GAC TTG CTC AGT GCT AAA GAA ATT GCT GTT
```

**2.** Procédé de préparation d'un vecteur de clonage selon la revendication 1, caractérisé en ce que l'ADNc comprend la séquence codante de nucléotides complète suivante :

```
                          ATG GCG GCA TCT GTC GAA AAC AGG CAG
TTC AGT CAC CTA GAA GCC GGT TTA TCC CGG TCT TTC AAG CCC
CGG TCT GAT TCC CCG GTT CGT GGC TGC AAC TTC CCT TCG CCC
AAC AGT ACT AAT TTC CAA AAG AAA CCA AAT TCC ACC ATT TAC
CTT GAT TAC TCG TCG AGT GAA GAC GAC GAT GAT GAT GAC GAA
AAA AAT GAG TAC CTT CAA ATG ATT AAA AAA GGG AAT TCA GAG
TTA GAG CCA TCT GTT CAT GAC ACT AGG GAC GAA GGT ACC GCT
GAT AAT TGG ATT GAA CGC AAC TTT TCC ATG ATT CGT CTC ACC
GGA AAG CAT CCA TTT AAC TCC GAA CCA CCG TTG AAC CGG CTC
ATG CAC CAC GGC TTT ATC ACA CCG GTC CCA CTT CAT TAC GTT
CGT AAC CAT GGA CCG GTT CCC AAG GGC ACG TGG GAT GAC TGG
ACC GTG GAA GTC ACG GGA CTA GTG AAG CGT CCT ATG AAA TTC
ACA ATG GAC CAG TTG GTT AAC GAA TTC CCT TGT AGA GAA TTG
CCC GTT ACG CTT GTT TGT GCT GGC AAT CGA AGG AAA GAA CAG
AAC ATG GTT AAA CAA ACC ATT GGT TTC AAC TGG GGC GCC GCT
GCC GTT TCA ACA ACG ATA TGG CGC GGG GTA CCC CTC CGC GCT
TTG CTA AAA CGG TGC GGT GTT TTT AGC AAG AAT AAA GGG GCG
CTT AAT GTT TGC TTC GAA GGA GCT GAT GTG TTG CCC GGA GGT
GGT GGT TCA AAG TAT GGA ACC AGC ATT AAG AAG GAA TTT GCA
ATG GAT CCA GCA CGA GAT ATC ATC GTA GCC TAC ATG CAG AAC
GGA GAA AAA TTG GCA CCC GAC CAC GGG TTT CCA GTA CGA ATG
ATA ATT CCA GGA TTC ATT GGA GGA AGA ATG GTG AAA TGG ATA
AAG AGG ATT ATA GTC ACC ACC CAA GAA TCA GAC AGC TAT TAT
CAT TTC AAG GAC AAT AGA GTT CTT CCT CCC CAT GTT GAT GCT
GAA CTT GCA AAT ACC GAA GCA TGG TGG TAC AAG CCA GAG TAT
ATC ATC AAT GAG CTT AAT ATT AAC TCT GTC ATT ACG ACG CCG
TGT CAT GAA GAA ATT TTG CCA ATT AAC GCC TGG ACG ACT CAG
CGA CCT TAC ACG TTG AGG GGC TAT TCT TAT TCT GGC GGA GGG
AAA AAA GTA ACG CGA GTA GAA GTG ACG TTG GAT GGA GGA GAA
ACA TGG CAA GTT AGC ACA CTA GAT CAC CCA GAG AAG CCC ACC
AAA TAT GGC AAG TAC TGG TGT TGG TGC TTT TGG TCA CTC GAG
GTT GAG GTG TTA GAC TTG CTC AGT GCT AAA GAA ATT GCT GTT
CGA GCT TGG GAT GAG ACC CTC AAT ACT CAA CCC GAG AAG CTT
ATT TGG AAC GTC ATG GGA ATG ATG AAT AAT TGC TGG TTC CGA
GTA AAG ATG AAT GTG TGC AAG CCT CAC AAG GGA GAG ATT GGA
ATA GTG TTT GAG CAT CCG ACT CAA CCT GGA AAC CAA TCA GGT
```

20

```
GGA TGG ATG GCG AAG GAG AGA CAT TTG GAG ATA TCA GCA GAG
GCA CCT CAA ACA CTA AAG AAG AGT ATC TCA ACT CCA TTC ATG
AAC ACA GCT TCC AAG ATG TAC TCC ATG TCC GAG GTC AGG AAA
CAC AGC TCT GCT GAC TCT GCT TGG ATC ATA GTC CAT GGT CAT
ATC TAT GAC GCC ACG CGT TTC TTG AAA GAT CAC CCT GGT GGG
ACT GAC AGC ATT CTC ATC AAT GCT GGC ACT GAT TGC ACT GAG
GAA TTT GAT GCA ATT CAT TCT GAT AAG GCT AAG AAG CTC TTG
GAG GAT TTC AGG ATT GGT GAA CTC ATA ACT ACT GGT TAC ACC
TCT GAC TCT CCT GGC AAC TCC GTG CAC GGA TCT TCT TCC TTC
AGC AGC TTT CTA GCA CCT ATT AAG GAA CTT GTT CCA GCG CAG
AGG AGT GTG GCC CTA ATT CCA AGA GAG AAA ATC CCA TGC AAA
CTC ATC GAC AAG CAA TCC ATC TCC CAT GAT GTT AGG AAA TTT
CGA TTT GCA TTG CCC TCT GAG GAT CAA GTC TTG GGC TTG CCT
GTT GGA AAA CAT ATC TTC CTC TGT GCC GTT ATT GAC GAT AAG
CTC TGC ATG CGC GCT TAC ACG CCT ACT AGC ACG ATC GAT GAG
GTG GGG TAC TTC GAG TTG GTT GTC AAG ATA TAC TTC AAA GGA
ATT CAC CCT AAA TTC CCC AAT GGA GGG CAA ATG TCA CAG TAT
CTT GAT TCT ATG CCG TTA GGG TCA TTT CTC GAC GTG AAA GGT
CCA TTA GGT CAC ATT GAA TAC CAA GGA AAG GGA AAT TTC TTA
GTT CAT GGC AAA CAG AAG TTT GCC AAG AAG TTG GCC ATG ATA
GCA GGT GGA ACA GGA ATA ACT CCA GTG TAT CAA GTC ATG CAG
GCA ATT CTG AAA GAT CCA GAA GAT GAC ACA GAA ATG TAT GTG
GTG TAT GCT AAC AGA ACA GAG GAT GAT ATT TTA CTT AAG GAA
GAG CTT GAT TCA TGG GCT GAG AAA ATT CCA GAG AGG GTT AAA
GTT TGG TAT GTG GTT CAG GAT TCT ATT AAA GAA GGA TGG AAG
TAC AGC ATT GGT TTT ATT ACA GAA GCC ATT TTG AGA GAA CAT
ATC CCT GAG CCA TCT CAC ACA ACA CTG GCT TTG GCT TGT GGA
CCA CCT CCT ATG ATT CAA TTT GCT GTT AAT CCA AAC TTG GAG
AAG ATG GGC TAT GAC ATT AAG GAT TCC TTA TTG GTG TTC TAA
```

## Claims

Claims for the following Contracting States : AT, BE, CH, LI, DE, FR, GB, GR, IT, LU, NL, SE

1.  A cloning vector comprising a complementary DNA of mRNA of the tobacco nitrate reductase in which the cDNA insert is of at least 1.6 kb and codes for are than 50% of the mRNA of the tobacco nitrate reductase, comprising the following coding sequence of nucleotides:

```
CCA CCT TCG CAT CAG ACC CTC AAT ACT CAA CCC CAG AAG CTT ATT TCG AAC CTC ATC CCA ATG
ATG AAT AAT TCC TCG TTC CCA GTA AAG ATG AAT GTC TCC AAG CCT CAC AAG CGA CAG ATT CCA
ATA GTG TTT CAG CAT CCG ACT CAA CCT CCA AAG CAA TCA CGT CCA TCC ATC CCG AAG CAG ACA
CAT TTG CAG ATA TCA GCA GAG CCA CCT CAA ACA CTA AAG AAG AGT ATC TCA ACT CCA TTC ATG
AAC ACA GCT TCC AAG ATG TAC TCC ATG TCC GAG GTC ACG AAA CAC AGC TCT GCT GAC TCT CCT
TCG ATC ATA GTC CAT CGT CAT ATC TAT GAC CCC ACG CCT TTC TTC AAA CAT CAC CCT CCT CCG
ACT CAC AGC ATT CTC ATC AAT GCT CCC ACT GAT TCC ACT CAG GAA TTT GAT CCA ATT CAT TCT
CAT AAG CCT AAG AAG CTC TTG CAG GAT TTC ACG ATT CGT GAA CTC ATA ACT ACT CCT TAC ACC
TCT CAC TCT CCT CCC AAC TCC CTG CAC CGA TCT TCT TCC TTC ACC ACC TTT CTA CCA CCT ATT
AAG CAA CTT CTT CCA CCG CAG ACC ACT CTC CCC CTA ATT CCA AGA CAU AAA ATC CCA TCC AAA
CTC ATC CAC AAG CAA TCC ATC TCC CAT CAT CTT ACG AAA TTT CGA TTT CCA TTC CCC TCT CAG
CAT CAA CTC TTC CCC TTC CCT CTT CCA AAA CAT ATC TTC CTC TCT CCC CTT ATT CAC CAT AAG
CTC TCC ATC CCC CCT TAC ACG CCT ACT ACC ACG ATC CAT CAG CTC CCG TAC TTC CAG TTC CTT
CTC AAG ATA TAC TTC AAA CCA ATT

CAA TTC CCT TCT ACA CAA TTG CCC CTT ACG CTT CTT TCT CCT CCC AAT CCA ACC AAA CAA CAG
AAC ATG CTT AAA CAA ACC ATT CCT TTC AAC TCC CCC CCC CCT CCC CTT TCA ACA ACC ATA TCG
CCC CCC CTA CCC CTC CCC CCT TTC CTA AAA CCC TCC CCT CTT TTT ACC AAC AAT AAA CCC CCC
CTT AAT CTT TCC TTC CAA CCA CCT CAT CTC TTC CCC CCA CCT CCT CCT TCA AAG TAT CCA ACC
ACC ATT AAC AAC CAA TTT CCA ATC CAT CCA CCA CCA CAT ATC ATC CTA CCC TAC ATC CAC AAC
CCA CAA AAA TTC CCA CCC CAC CAC CCC TTT CCA CTA CCA ATC ATA ATT CCA CCA TTC ATT CCA
CCA ACA ATC CTC AAA TCC ATA AAC ACC ATT ATA CTC ACC ACC CAA CAA TCA CAC ACC TAT TAT
CAT TTC AAC CAC AAT ACA CTT CTT CCT CCC CAT CTT CAT CCT CAA CTT CCA AAT ACC CAA CCA
TCC TCC TAC AAC CCA CAC TAT ATC ATC AAT CAC CTT AAT ATT AAC TCT CTC ATT ACC ACC CCC
TCT CAT CAA CAA ATT TTC CCA ATT AAC TCC TCC ACC ACT CAC CTA CCT TAC ACC TTC ACC CCC
TAT TCT TAT TCT CCC CCA CCC AAA AAA CTA ACC CCA CTA CAA CTC ACC TTC CAT CCA CCA CAA
ACA TCC CAA CTT ACC ACA CTA CAT CAC CCA CAC AAC CCC ACC AAA TAT CCC AAC TAC TCC TCT
TCC TCC TTT TCC TCA CTC CAC CTT CAC CTC TTA CAC TTC CTC ACT CCT AAA CAA ATT CCT CTT
```

**2.** A cloning vector according to claim 1 characterised in that it comprises the following complete coding sequence of nucleotides:

```
                                    ATG GCG GCA TCT GTC GAA AAC AGG CAG
TTC AGT CAC CTA GAA GCC GGT TTA TCC CGG TCT TTC AAG CCC
CGG TCT GAT TCC CCG GTT CGT GGC TGC AAC TTC CCT TCG CCC
AAC AGT ACT AAT TTC CAA AAG AAA CCA AAT TCC ACC ATT TAC
CTT GAT TAC TCG TCG AGT GAA GAC GAC GAT GAT GAT GAC GAA
AAA AAT GAG TAC CTT CAA ATG ATT AAA AAA GGG AAT TCA GAG
TTA GAG CCA TCT GTT CAT GAC ACT AGG GAC GAA GGT ACC GCT
GAT AAT TGG ATT GAA CGC AAC TTT TCC ATG ATT CGT CTC ACC
GGA AAG CAT CCA TTT AAC TCC GAA CCA CCG TTG AAC CGG CTC
ATG CAC CAC GGC TTT ATC ACA CCG GTC CCA CTT CAT TAC GTT
CGT AAC CAT GGA CCG GTT CCC AAG GGC ACG TGG GAT GAC TGG
ACC GTG GAA GTC ACG GGA CTA GTG AAG CGT CCT ATG AAA TTC
ACA ATG GAC CAG TTG GTT AAC GAA TTC CCT TGT AGA GAA TTG
CCC GTT ACG CTT GTT TGT GCT GGC AAT CGA AGG AAA GAA CAG
AAC ATG GTT AAA CAA ACC ATT GGT TTC AAC TGG GGC GCC GCT
GCC GTT TCA ACA ACG ATA TGG CGC GGG GTA CCC CTC CGC GCT
TTG CTA AAA CGG TGC GGT GTT TTT AGC AAG AAT AAA GGG GCG
CTT AAT GTT TGC TTC GAA GGA GCT GAT GTG TTG CCC GGA GGT
GGT GGT TCA AAG TAT GGA ACC AGC ATT AAG AAG GAA TTT GCA
ATG GAT CCA GCA CGA GAT ATC ATC GTA GCC TAC ATG CAG AAC
GGA GAA AAA TTG GCA CCC GAC CAC GGG TTT CCA GTA CGA ATG
ATA ATT CCA GGA TTC ATT GGA GGA AGA ATG GTG AAA TGG ATA
AAG AGG ATT ATA GTC ACC ACC CAA GAA TCA GAC AGC TAT TAT
CAT TTC AAG GAC AAT AGA GTT CTT CCT CCC CAT GTT GAT GCT
GAA CTT GCA AAT ACC GAA GCA TGG TGG TAC AAG CCA GAG TAT
ATC ATC AAT GAG CTT AAT ATT AAC TCT GTC ATT ACG ACG CCG
TGT CAT GAA GAA ATT TTG CCA ATT AAC GCC TGG ACG ACT CAG
CGA CCT TAC ACG TTG AGG GGC TAT TCT TAT TCT GGC GGA GGG
AAA AAA GTA ACG CGA GTA GAA GTG ACG TTG GAT GGA GGA GAA
ACA TGG CAA GTT AGC ACA CTA GAT CAC CCA GAG AAG CCC ACC
AAA TAT GGC AAG TAC TGG TGT TGG TGC TTT TGG TCA CTC GAG
GTT GAG GTG TTA GAC TTG CTC AGT GCT AAA GAA ATT GCT GTT
CGA GCT TGG GAT GAG ACC CTC AAT ACT CAA CCC GAG AAG CTT
ATT TGG AAC GTC ATG GGA ATG ATG AAT AAT TGC TGG TTC CGA
GTA AAG ATG AAT GTG TGC AAG CCT CAC AAG GGA GAG ATT GGA
ATA GTG TTT GAG CAT CCG ACT CAA CCT GGA AAC CAA TCA GGT
```

```
GGA TGG ATG GCG AAG GAG AGA CAT TTG GAG ATA TCA GCA GAG
GCA CCT CAA ACA CTA AAG AAG AGT ATC TCA ACT CCA TTC ATG
AAC ACA GCT TCC AAG ATG TAC TCC ATG TCC GAG GTC AGG AAA
CAC AGC TCT GCT GAC TCT GCT TGG ATC ATA GTC CAT GGT CAT
ATC TAT GAC GCC ACG CGT TTC TTG AAA GAT CAC CCT GGT GGG
ACT GAC AGC ATT CTC ATC AAT GCT GGC ACT GAT TGC ACT GAG
GAA TTT GAT GCA ATT CAT TCT GAT AAG GCT AAG AAG CTC TTG
GAG GAT TTC AGG ATT GGT GAA CTC ATA ACT ACT GGT TAC ACC
TCT GAC TCT CCT GGC AAC TCC GTG CAC GGA TCT TCT TCC TTC
AGC AGC TTT CTA GCA CCT ATT AAG GAA CTT GTT CCA GCG CAG
AGG AGT GTG GCC CTA ATT CCA AGA GAG AAA ATC CCA TGC AAA
CTC ATC GAC AAG CAA TCC ATC TCC CAT GAT GTT AGG AAA TTT
CGA TTT GCA TTG CCC TCT GAG GAT CAA GTC TTG GGC TTG CCT
GTT GGA AAA CAT ATC TTC CTC TGT GCC GTT ATT GAC GAT AAG
CTC TGC ATG CGC GCT TAC ACG CCT ACT AGC ACG ATC GAT GAG
GTG GGG TAC TTC GAG TTG GTT GTC AAG ATA TAC TTC AAA GGA
ATT CAC CCT AAA TTC CCC AAT GGA GGG CAA ATG TCA CAG TAT
CTT GAT TCT ATG CCG TTA GGG TCA TTT CTC GAC GTG AAA GGT
CCA TTA GGT CAC ATT GAA TAC CAA GGA AAG GGA AAT TTC TTA
GTT CAT GGC AAA CAG AAG TTT GCC AAG AAG TTG GCC ATG ATA
GCA GGT GGA ACA GGA ATA ACT CCA GTG TAT CAA GTC ATG CAG
GCA ATT CTG AAA GAT CCA GAA GAT GAC ACA GAA ATG TAT GTG
GTG TAT GCT AAC AGA ACA GAG GAT GAT ATT TTA CTT AAG GAA
GAG CTT GAT TCA TGG GCT GAG AAA ATT CCA GAG AGG GTT AAA
GTT TGG TAT GTG GTT CAG GAT TCT ATT AAA GAA GGA TGG AAG
TAC AGC ATT GGT TTT ATT ACA GAA GCC ATT TTG AGA GAA CAT
ATC CCT GAG CCA TCT CAC ACA ACA CTG GCT TTG GCT TGT GGA
CCA CCT CCT ATG ATT CAA TTT GCT GTT AAT CCA AAC TTG GAG
AAG ATG GGC TAT GAC ATT AAG GAT TCC TTA TTG GTG TTC TAA
```

**Claims for the the following Contracting State : ES**

1.  A process for preparing a cloning vector comprising a complementary DNA of mRNA of the tobacco nitrate reductase, characterised by inserting a cDNA of at least 1.6 kb which codes for are than 50% of the mRNA of the tobacco nitrate reductase, comprising the following coding sequence of nucleotides:

```
CCA GGT TCG GAT GAC ACC CTC AAT ACT CAA CCC CAG AAG CTT ATT TCG AAC CTC ATC CCA ATG
ATG AAT AAT TGC TCG TTC CGA GTA AAG ATG AAT GTG TCC AAG GCT CAC AAG CCA CAG ATT CCA
ATA GTG TTT CAG CAT CCG ACT CAA CCT CCA AAG CAA TCA CGT CGA TCG ATC GCC AAG CAG ACA
CAT TTG CAG ATA TCA CCA CAG GCA CCT CAA ACA CTA AAG AAG AGT ATC TCA ACT CCA TTC ATG
AAC ACA GCT TCC AAG ATG TAC TCC ATG TCC CAG GTC ACG AAA CAC ACC TCT GCT CAC TCT CCT
TCG ATC ATA GTC CAT GGT CAT ATC TAT CAC CCC ACC CGT TTC TTG AAA GAT CAC CCT CCT CCG
ACT CAC ACC ATT CTC ATC AAT GCT CCC ACT GAT TCC ACT CAG GAA TTT GAT GCA ATT CAT TCT
CAT AAG CCT AAG AAG CTC TTC GAG GAT TTC ACG ATT CGT GAA CTC ATA ACT ACT CCT TAC ACC
TCT CAG TCT CCT CGG AAG TCC GTG CAC CGA TCT TCT TCG TTC AGG AGG TTT CTA GCA CCT ATT
AAG CAA CTT GTT CCA CCG CAG ACG AGT GTG GCC GTA ATT CCA AGA CAU AAA ATC CCA TCC AAA
CTC ATC CAC AAG CAA TCC ATC TCC CAT GAT GTT ACG AAA TTT CGA TTT CCA TTG CCC TCT CAG
GAT CAA GTC TTG CGG TTG CGT GTT GGA AAA GAT ATG TTC CTC TCT GCC CTT ATT CAC GAT AAG
CTC TCG ATC CCC GCT TAC ACG CCT ACT AGG ACG ATC GAT CAG CTC GCG TAC TTC CAG TTC GTT
GTC AAG ATA TAC TTC AAA GGA ATT

CAA TTC CCT TCT AGA GAA TTG GGG GTT ACG CTT GTT TGT GGT CGG AAT CCA ACG AAA CAA CAG
AAC ATG GTT AAA CAA ACC ATT GGT TTC AAG TGG GGG GGG GGT GGG GTT TCA ACA ACG ATA TGG
CCC GGG GTA CGC CTC CGG GGT TTG CTA AAA GGG TCG GGT GTT TTT AGG AAG AAT AAA GGG GGG
GTT AAT GTT GGG TTC GAA GGA GGT GAT GTG TTG CGG GGA GGT GGT GGT TCA AAG TAT GGA AGG
AGG ATT AAG AAG GAA TTT GGA ATG GAT GGA GGA GGA GAT ATG ATG GTA GGG TAG ATG GAG AAG
GGA GAA AAA TTG GGA GGG GAG GAG GGG TTT GGA GTA GGA ATG ATA ATT GGA GGA TTG ATT GGA
GGA AGA ATG GTG AAA TGG ATA AAG AGG ATT ATA GTG AGG AGG GAA GAA TGA GAG AGG TAT TAT
GAT TTG AAG GAG AAT AGA GTT GTT GGT GGG GAT GTT GAT GGT GAA GTT GGA AAT AGG GAA GGA
TGG TGG TAG AAG GGA GAG TAT ATG ATG AAT GAG GTT AAT ATT AAG TGT GTG ATT AGG AGG GGG
TGT GAT GAA GAA ATT TTG GGA ATT AAG GGG TGG AGG AGT GAG GUA GGT TAG AGG TTG AGG GGG
TAT TGT TAT TGT GGG GGA GGG AAA AAA GTA AGG GGA GTA GAA GTG AGG TTG GAT GGA GGA GAA
AGA TGG GAA GTT AGG AGA GTA GAT GAG GGA GAG AAG GGG AGG AAA TAT GGG AAG TAG TGG TGT
TGG TGG TTT TGG TGA GTG GAG GTT GAG GTG TTA GAG TTG GTG AGT GGT AAA GAA ATT GGT GTT
```

2. A process for preparing a cloning vector according to claim 1 characterised in that the cDNA comprises the following complete coding sequence of nucleotides:

```
                                    ATG GCG GCA TCT GTC GAA AAC AGG CAG
            TTC AGT CAC CTA GAA GCC GGT TTA TCC CGG TCT TTC AAG CCC
            CGG TCT GAT TCC CCG GTT CGT GGC TGC AAC TTC CCT TCG CCC
            AAC AGT ACT AAT TTC CAA AAG AAA CCA AAT TCC ACC ATT TAC
            CTT GAT TAC TCG TCG AGT GAA GAC GAC GAT GAT GAT GAC GAA
            AAA AAT GAG TAC CTT CAA ATG ATT AAA AAA GGG AAT TCA GAG
            TTA GAG CCA TCT GTT CAT GAC ACT AGG GAC GAA GGT ACC GCT
            GAT AAT TGG ATT GAA CGC AAC TTT TCC ATG ATT CGT CTC ACC
            GGA AAG CAT CCA TTT AAC TCC GAA CCA CCG TTG AAC CGG CTC
            ATG CAC CAC GGC TTT ATC ACA CCG GTC CCA CTT CAT TAC GTT
            CGT AAC CAT GGA CCG GTT CCC AAG GGC ACG TGG GAT GAC TGG
            ACC GTG GAA GTC ACG GGA CTA GTG AAG CGT CCT ATG AAA TTC
            ACA ATG GAC CAG TTG GTT AAC GAA TTC CCT TGT AGA GAA TTG
            CCC GTT ACG CTT GTT TGT GCT GGC AAT CGA AGG AAA GAA CAG
            AAC ATG GTT AAA CAA ACC ATT GGT TTC AAC TGG GGC GCC GCT
            GCC GTT TCA ACA ACG ATA TGG CGC GGG GTA CCC CTC CGC GCT
            TTG CTA AAA CGG TGC GGT GTT TTT AGC AAG AAT AAA GGG GCG
            CTT AAT GTT TGC TTC GAA GGA GCT GAT GTG TTG CCC GGA GGT
            GGT GGT TCA AAG TAT GGA ACC AGC ATT AAG AAG GAA TTT GCA
            ATG GAT CCA GCA CGA GAT ATC ATC GTA GCC TAC ATG CAG AAC
            GGA GAA AAA TTG GCA CCC GAC CAC GGG TTT CCA GTA CGA ATG
            ATA ATT CCA GGA TTC ATT GGA GGA AGA ATG GTG AAA TGG ATA
            AAG AGG ATT ATA GTC ACC ACC CAA GAA TCA GAC AGC TAT TAT
            CAT TTC AAG GAC AAT AGA GTT CTT CCT CCC CAT GTT GAT GCT
            GAA CTT GCA AAT ACC GAA GCA TGG TGG TAC AAG CCA GAG TAT
            ATC ATC AAT GAG CTT AAT ATT AAC TCT GTC ATT ACG ACG CCG
            TGT CAT GAA GAA ATT TTG CCA ATT AAC GCC TGG ACG ACT CAG
            CGA CCT TAC ACG TTG AGG GGC TAT TCT TAT TCT GGC GGA GGG
            AAA AAA GTA ACG CGA GTA GAA GTG ACG TTG GAT GGA GGA GAA
            ACA TGG CAA GTT AGC ACA CTA GAT CAC CCA GAG AAG CCC ACC
            AAA TAT GGC AAG TAC TGG TGT TGG TGC TTT TGG TCA CTC GAG
            GTT GAG GTG TTA GAC TTG CTC AGT GCT AAA GAA ATT GCT GTT
            CGA GCT TGG GAT GAG ACC CTC AAT ACT CAA CCC GAG AAG CTT
            ATT TGG AAC GTC ATG GGA ATG ATG AAT AAT TGC TGG TTC CGA
            GTA AAG ATG AAT GTG TGC AAG CCT CAC AAG GGA GAG ATT GGA
            ATA GTG TTT GAG CAT CCG ACT CAA CCT GGA AAC CAA TCA GGT
```

```
GGA TGG ATG GCG AAG GAG AGA CAT TTG GAG ATA TCA GCA GAG
GCA CCT CAA ACA CTA AAG AAG AGT ATC TCA ACT CCA TTC ATG
AAC ACA GCT TCC AAG ATG TAC TCC ATG TCC GAG GTC AGG AAA
CAC AGC TCT GCT GAC TCT GCT TGG ATC ATA GTC CAT GGT CAT
ATC TAT GAC GCC ACG CGT TTC TTG AAA GAT CAC CCT GGT GGG
ACT GAC AGC ATT CTC ATC AAT GCT GGC ACT GAT TGC ACT GAG
GAA TTT GAT GCA ATT CAT TCT GAT AAG GCT AAG AAG CTC TTG
GAG GAT TTC AGG ATT GGT GAA CTC ATA ACT ACT GGT TAC ACC
TCT GAC TCT CCT GGC AAC TCC GTG CAC GGA TCT TCT TCC TTC
AGC AGC TTT CTA GCA CCT ATT AAG GAA CTT GTT CCA GCG CAG
AGG AGT GTG GCC CTA ATT CCA AGA GAG AAA ATC CCA TGC AAA
CTC ATC GAC AAG CAA TCC ATC TCC CAT GAT GTT AGG AAA TTT
CGA TTT GCA TTG CCC TCT GAG GAT CAA GTC TTG GGC TTG CCT
GTT GGA AAA CAT ATC TTC CTC TGT GCC GTT ATT GAC GAT AAG
CTC TGC ATG CGC GCT TAC ACG CCT ACT AGC ACG ATC GAT GAG
GTG GGG TAC TTC GAG TTG GTT GTC AAG ATA TAC TTC AAA GGA
ATT CAC CCT AAA TTC CCC AAT GGA GGG CAA ATG TCA CAG TAT
CTT GAT TCT ATG CCG TTA GGG TCA TTT CTC GAC GTG AAA GGT
CCA TTA GGT CAC ATT GAA TAC CAA GGA AAG GGA AAT TTC TTA
GTT CAT GGC AAA CAG AAG TTT GCC AAG AAG TTG GCC ATG ATA
GCA GGT GGA ACA GGA ATA ACT CCA GTG TAT CAA GTC ATG CAG
GCA ATT CTG AAA GAT CCA GAA GAT GAC ACA GAA ATG TAT GTG
GTG TAT GCT AAC AGA ACA GAG GAT GAT ATT TTA CTT AAG GAA
GAG CTT GAT TCA TGG GCT GAG AAA ATT CCA GAG AGG GTT AAA
GTT TGG TAT GTG GTT CAG GAT TCT ATT AAA GAA GGA TGG AAG
TAC AGC ATT GGT TTT ATT ACA GAA GCC ATT TTG AGA GAA CAT
ATC CCT GAG CCA TCT CAC ACA ACA CTG GCT TTG GCT TGT GGA
CCA CCT CCT ATG ATT CAA TTT GCT GTT AAT CCA AAC TTG GAG
AAG ATG GGC TAT GAC ATT AAG GAT TCC TTA TTG GTG TTC TAA
```

## Patentansprüche
**Patentansprüche für folgende Vertragsstaaten : AT, BE, CH, LI, DE, FR, GB, GR, IT, LU, NL, SE**

1. Klonierungsvektor, der eine DNA aufweist, die zur mRNA der Nitrat-Reduktase von Tabak komplementär ist, worin das cDNA-Insert mindestens 1,6 kb beträgt und für mehr als 50 % der mRNA der Nitrat-Reduktase von Tabak Codiert, der die folgende codierende Sequenz von Nucleotiden aufweist:

```
CCA GCT TGG GAT GAG ACC CTC AAT ACT CAA CCC GAG AAG CTT ATT TCG AAC GTC ATC GGA ATG
ATG AAT AAT TGG TGG TTC CGA GTA AAG ATG AAT GTG TGC AAG CCT CAC AAG GGA GAG ATT CCA
ATA GTG TTT GAG CAT CCG ACT CAA CCT GGA AAC CAA TCA GGT GCA TGG ATC GCG AAG GAG ACA
CAT TTG GAG ATA TCA GCA GAG GCA CCT CAA ACA CTA AAG AAG AGT ATC TCA ACT CCA TTC ATG
AAG ACA GCT TCC AAG ATG TAC TCC ATG TCC GAG GTC AGG AAA CAC AGC TCT GCT GAC TCT GCT
TGG ATC ATA GTC CAT GGT CAT ATC TAT GAC CCC ACG CGT TTC TTC AAA GAT CAC CCT CGT GCC
ACT GAC AGC ATT CTC ATC AAT GCT GGC ACT GAT TGC ACT GAG GAA TTT GAT GCA ATT CAT TCT
CAT AAG GCT AAG AAG CTC TTG GAG GAT TTC ACG ATT GGT GAA CTC ATA ACT ACT GGT TAC ACC
TGT GAC TCT CCT GGC AAG TCC GTG CAC GGA TCT TCT TCC TTC AGC AGC TTT CTA GCA CCT ATT
AAG GAA CTT GTT CCA GCG CAC AGG AGT GTG GCC CTA ATT CCA AGA GAG AAA ATC CCA TCC AAA
CTC ATC GAC AAG CAA TCC ATC TCC CAT GAT GTT AGG AAA TTT CGA TTT GCA TTG CCC TCT GAG
GAT CAA GTC TTG GGC TTG CCT GTT GGA AAA CAT ATC TTC CTC TGT GCC GTT ATT GAC GAT AAG
CTC TGC ATC CGC GCT TAC ACG CCT ACT AGC ACG ATC GAT GAG GTG GGG TAC TTC GAG TTG GTT
CTC AAG ATA TAC TTC AAA GGA ATT
```

```
CAA TTC CCT TCT ACA GAA TTG CCC GTT ACG CTT GTT TGT GGT GGC AAT GGA AGG AAA GAA GAG
AAC ATG GTT AAA CAA ACC ATT GGT TTC AAG TGG GGC GGC GGT GCC GTT TCA ACA ACG ATA TGG
GGG CGG GTA GGC GTC GGC GGT TTG CTA AAA GGG TGG GGT GTT TTT ACG AAG AAT AAA GGG CCG
CTT AAT GTT TGG TTC GAA GGA GGT GAT GTG TTG GGG GGA GGT GGT GGT TCA AAG TAT GGA AGG
AGC ATT AAG AAG CAA TTT GCA ATG GAT CCA GCA GGA GAT ATC ATC GTA GGG TAC ATG GAG AAC
GGA GAA AAA TTG GCA GGG GAG GAG GGG TTT CCA GTA GGA ATG ATA ATT GGA GGA TTC ATT GGA
GGA AGA ATG GTG AAA TGG ATA AAG AGG ATT ATA GTC AGG AGG GAA GAG TGA GAG AGG TAT TAT
GAT TTG AAG GAG AAT AGA GTT GTT GGT GGG GAT GTT GAG GGT GAA GTT GGA AAT GGG AAG GGA
TGG TGG TAG AAG GGA GAG TAT ATG ATG AAT GAG GTT AAT ATT AAG TGT GTG ATT AGG AGG GGG
TGT GAT GAA ATT TTG GGA ATT AAG GGG TGG AGG AGT GAG GGA GGT TAG AGG TTG AGG GGG
TAT TGT TAT TGT GGG GGA GGG AAA AAA GTA AGG GGA GTA GAA GTG AGG TTG GAT GGA GGA GAA
ACA TGG GAA GTT AGG AGA GTA GAT GAG GGA GAG AAG GGG AGG AAA TAT GGG AAG TAG TGG TGT
TGG TGG TTT TGG TGA GTG GAG GTT GAG GTG TTA GAG TTG GTG AGT GGT AAA GAA ATT GGT GTT
```

**2.** Klonierungsvektor nach Anspruch 1, dadurch gekennzeichnet, daß er die folgende vollständige codierende Sequenz von Nucleotiden aufweist:

```
                            ATG GCG GCA TCT GTC GAA AAC AGG CAG
TTC AGT CAC CTA GAA GCC GGT TTA TCC CGG TCT TTC AAG CCC
CGG TCT GAT TCC CCG GTT CGT GGC TGC AAC TTC CCT TCG CCC
AAC AGT ACT AAT TTC CAA AAG AAA CCA AAT TCC ACC ATT TAC
CTT GAT TAC TCG TCG AGT GAA GAC GAC GAT GAT GAT GAC GAA
AAA AAT GAG TAC CTT CAA ATG ATT AAA AAA GGG AAT TCA GAG
TTA GAG CCA TCT GTT CAT GAC ACT AGG GAC GAA GGT ACC GCT
GAT AAT TGG ATT GAA CGC AAC TTT TCC ATG ATT CGT CTC ACC
GGA AAG CAT CCA TTT AAC TCC GAA CCA CCG TTG AAC CGG CTC
ATG CAC CAC GGC TTT ATC ACA CCG GTC CCA CTT CAT TAC GTT
CGT AAC CAT GGA CCG GTT CCC AAG GGC ACG TGG GAT GAC TGG
ACC GTG GAA GTC ACG GGA CTA GTG AAG CGT CCT ATG AAA TTC
ACA ATG GAC CAG TTG GTT AAC GAA TTC CCT TGT AGA GAA TTG
CCC GTT ACG CTT GTT TGT GCT GGC AAT CGA AGG AAA GAA CAG
AAC ATG GTT AAA CAA ACC ATT GGT TTC AAC TGG GGC GCC GCT
GCC GTT TCA ACA ACG ATA TGG CGC GGG GTA CCC CTC CGC GCT
TTG CTA AAA CGG TGC GGT GTT TTT AGC AAG AAT AAA GGG GCG
CTT AAT GTT TGC TTC GAA GGA GCT GAT GTG TTG CCC GGA GGT
GGT GGT TCA AAG TAT GGA ACC AGC ATT AAG AAG GAA TTT GCA
ATG GAT CCA GCA CGA GAT ATC ATC GTA GCC TAC ATG CAG AAC
GGA GAA AAA TTG GCA CCC GAC CAC GGG TTT CCA GTA CGA ATG
ATA ATT CCA GGA TTC ATT GGA GGA AGA ATG GTG AAA TGG ATA
AAG AGG ATT ATA GTC ACC ACC CAA GAA TCA GAC AGC TAT TAT
CAT TTC AAG GAC AAT AGA GTT CTT CCT CCC CAT GTT GAT GCT
GAA CTT GCA AAT ACC GAA GCA TGG TGG TAC AAG CCA GAG TAT
ATC ATC AAT GAG CTT AAT ATT AAC TCT GTC ATT ACG ACG CCG
TGT CAT GAA GAA ATT TTG CCA ATT AAC GCC TGG ACG ACT CAG
CGA CCT TAC ACG TTG AGG GGC TAT TCT TAT TCT GGC GGA GGG
AAA AAA GTA ACG CGA GTA GAA GTG ACG TTG GAT GGA GGA GAA
ACA TGG CAA GTT AGC ACA CTA GAT CAC CCA GAG AAG CCC ACC
AAA TAT GGC AAG TAC TGG TGT TGG TGC TTT TGG TCA CTC GAG
GTT GAG GTG TTA GAC TTG CTC AGT GCT AAA GAA ATT GCT GTT
CGA GCT TGG GAT GAG ACC CTC AAT ACT CAA CCC GAG AAG CTT
```

```
ATT TGG AAC GTC ATG GGA ATG ATG AAT AAT TGC TGG TTC CGA
GTA AAG ATG AAT GTG TGC AAG CCT CAC AAG GGA GAG ATT GGA
ATA GTG TTT GAG CAT CCG ACT CAA CCT GGA AAC CAA TCA GGT
GGA TGG ATG GCG AAG GAG AGA CAT TTG GAG ATA TCA GCA GAG
GCA CCT CAA ACA CTA AAG AAG AGT ATC TCA ACT CCA TTC ATG
AAC ACA GCT TCC AAG ATG TAC TCC ATG TCC GAG GTC AGG AAA
CAC AGC TCT GCT GAC TCT GCT TGG ATC ATA GTC CAT GGT CAT
ATC TAT GAC GCC ACG CGT TTC TTG AAA GAT CAC CCT GGT GGG
ACT GAC AGC ATT CTC ATC AAT GCT GGC ACT GAT TGC ACT GAG
GAA TTT GAT GCA ATT CAT TCT GAT AAG GCT AAG AAG CTC TTG
GAG GAT TTC AGG ATT GGT GAA CTC ATA ACT ACT GGT TAC ACC
TCT GAC TCT CCT GGC AAC TCC GTG CAC GGA TCT TCT TCC TTC
AGC AGC TTT CTA GCA CCT ATT AAG GAA CTT GTT CCA GCG CAG
AGG AGT GTG GCC CTA ATT CCA AGA GAG AAA ATC CCA TGC AAA
CTC ATC GAC AAG CAA TCC ATC TCC CAT GAT GTT AGG AAA TTT
CGA TTT GCA TTG CCC TCT GAG GAT CAA GTC TTG GGC TTG CCT
GTT GGA AAA CAT ATC TTC CTC TGT GCC GTT ATT GAC GAT AAG
CTC TGC ATG CGC GCT TAC ACG CCT ACT AGC ACG ATC GAT GAG
GTG GGG TAC TTC GAG TTG GTT GTC AAG ATA TAC TTC AAA GGA
ATT CAC CCT AAA TTC CCC AAT GGA GGG CAA ATG TCA CAG TAT
CTT GAT TCT ATG CCG TTA GGG TCA TTT CTC GAC GTG AAA GGT
CCA TTA GGT CAC ATT GAA TAC CAA GGA AAG GGA AAT TTC TTA
GTT CAT GGC AAA CAG AAG TTT GCC AAG AAG TTG GCC ATG ATA
GCA GGT GGA ACA GGA ATA ACT CCA GTG TAT CAA GTC ATG CAG
GCA ATT CTG AAA GAT CCA GAA GAT GAC ACA GAA ATG TAT GTG
GTG TAT GCT AAC AGA ACA GAG GAT GAT ATT TTA CTT AAG GAA
GAG CTT GAT TCA TGG GCT GAG AAA ATT CCA GAG AGG GTT AAA
GTT TGG TAT GTG GTT CAG GAT TCT ATT AAA GAA GGA TGG AAG
TAC AGC ATT GGT TTT ATT ACA GAA GCC ATT TTG AGA GAA CAT
ATC CCT GAG CCA TCT CAC ACA ACA CTG GCT TTG GCT TGT GGA
CCA CCT CCT ATG ATT CAA TTT GCT GTT AAT CCA AAC TTG GAG
AAG ATG GGC TAT GAC ATT AAG GAT TCC TTA TTG GTG TTC TAA
```

**Patentansprüche für folgenden Vertragsstaat : ES**

1. Verfahren zur Herstellung eines Klonierungsvektors, der eine DNA aufweist, die zur mRNA der Nitrat-Reduktase von Tabak komplementär ist, dadurch gekennzeichnet, daß man eine cDNA mit mindestens 1,6 kb inseriert, die für mehr als 50 % der mRNA der Nitrat-Reduktase von Tabak codiert, der die folgende codierende Sequenz von Nucleotiden aufweist:

CCA CCT TCC CAT CAG ACC CTC AAT ACT CAA CCC CAG AAG CTT ATT TCC AAC CTC ATG CCA ATG
ATG AAT AAT TCC TCC TTC CCA GTA AAG ATG AAT GTG TCC AAG CCT CAC AAG CCA CAG ATT CCA
ATA GTG TTT CAG CAT CCG ACT CAA CCT CCA AAG CAA TCA CCT CCA TCC ATC CCC AAG CAG ACA
CAT TTC CAG ATA TCA CCA GAG CCA CCT CAA ACA CTA AAG AAG ACT ATC TCA ACT CCA TTC ATC
AAC ACA CCT TCC AAG ATC TAC TCC ATC TCC CAG CTC ACG AAA CAC ACC TCT CCT CAC TCT CCT
TCC ATC ATA CTC CAT CCT CAT ATC TAT CAC CCC ACG CCT TTC TTC AAA CAT CAC CCT CCT CCC
ACT CAC ACC ATT CTC ATC AAT CCT CCC ACT CAT TCC ACT CAG CAA TTT CAT CCA ATT CAT TCT
CAT AAG CCT AAG AAG CTC TTG CAG CAT TTC ACG ATT CCT CAA CTC ATA ACT ACT CCT TAC ACC
TCT CAG TCT CCT CCC AAG TCC GTG CAC CCA TCT TCT TCC TTC ACC ACC TTT CTA CCA CCT ATT
AAG CAA CTT CTT CCA CCG CAG ACG AGT CTC CCC CTA ATT CCA ACA CAU AAA ATC CCA TCC AAA
CTC ATC CAC AAG CAA TCC ATC TCC CAT CAT CTT ACG AAA TTT CCA TTT CCA TTC CCC TCT CAG
CAT CAA CTC TTG CCC TTG CCT CTT CCA AAA CAT ATC TTC CTC TCT CCC CTT ATT CAC CAT AAG
CTC TCC ATC CCC CCT TAC ACG CCT ACT ACC ACG ATC CAT CAG CTC CCG TAC TTC CAG TTG CTT
CTC AAG ATA TAC TTC AAA CCA ATT

CAA TTC CCT TCT ACA CAA TTG CCC CTT ACG CTT CTT TCT CCT CCC AAT CCA ACC AAA CAA CAG
AAC ATC CTT AAA CAA ACC ATT CCT TTC AAG TCC CCC CCC CCT CCC CTT TCA ACA ACG ATA TCC
CCC CCC CTA CCC CTC CCC CCT TTC CTA AAA CCC TCC CCT CTT TTT ACC AAC AAT AAA CCC CCC
CTT AAT CTT TCC TTC CAA CCA CCT CAT CTC TTC CCC CCA CCT CCT CCT TCA AAC TAT CCA ACC
ACC ATT AAG AAG CAA TTT CCA ATC CAT CCA CCA CCA CAT ATC ATC CTA CCC TAC ATC CAC AAC
CCA CAA AAA TTC CCA CCC CAC CAC CCC TTT CCA CTA CCA ATC ATA ATT CCA CCA TTC ATT CCA
CCA ACA ATC CTC AAA TCC ATA AAG ACC ATT ATA CTC ACC ACC CAA CAA TCA CAC ACC TAT TAT
CAT TTC AAG CAC AAT ACA CTT CTT CCT CCC CAT CTT CAT CCT CAA CTT CCA AAT ACC CAA CCA
TCC TCC TAC AAG CCA CAG TAT ATC ATC AAT CAG CTT AAT ATT AAG TCT CTC ATT ACG ACG CCG
TCT CAT CAA CAA ATT TTG CCA ATT AAG TCC TCC ACG ACT CAG CTA CCT TAC ACG TTG ACG CCC
TAT TCT TAT TCT CCC CCA CCC AAA AAA CTA ACG CCA CTA CAA CTC ACG TTC CAT CCA CCA CAA
ACA TCC CAA CTT ACC ACA CTA CAT CAC CCA CAG AAG CCC ACC AAA TAT CCC AAG TAC TCC TCT
TCC TCC TTT TCC TCA CTC CAG CTT CAG CTG TTA CAC TTG CTC ACT CCT AAA CAA ATT CCT CTT

**2.** Verfahren zur Herstellung eines Klonierungsvektors nach Anspruch 1, dadurch gekennzeichnet, daß die cDNA die folgende vollständige codierende Sequenz von Nucleotiden aufweist:

```
                              ATG GCG GCA TCT GTC GAA AAC AGG CAG
        TTC AGT CAC CTA GAA GCC GGT TTA TCC CGG TCT TTC AAG CCC
        CGG TCT GAT TCC CCG GTT CGT GGC TGC AAC TTC CCT TCG CCC
        AAC AGT ACT AAT TTC CAA AAG AAA CCA AAT TCC ACC ATT TAC
        CTT GAT TAC TCG TCG AGT GAA GAC GAC GAT GAT GAT GAC GAA
        AAA AAT GAG TAC CTT CAA ATG ATT AAA AAA GGG AAT TCA GAG
        TTA GAG CCA TCT GTT CAT GAC ACT AGG GAC GAA GGT ACC GCT
        GAT AAT TGG ATT GAA CGC AAC TTT TCC ATG ATT CGT CTC ACC
        GGA AAG CAT CCA TTT AAC TCC GAA CCA CCG TTG AAC CGG CTC
        ATG CAC CAC GGC TTT ATC ACA CCG GTC CCA CTT CAT TAC GTT
        CGT AAC CAT GGA CCG GTT CCC AAG GGC ACG TGG GAT GAC TGG
        ACC GTG GAA GTC ACG GGA CTA GTG AAG CGT CCT ATG AAA TTC
        ACA ATG GAC CAG TTG GTT AAC GAA TTC CCT TGT AGA GAA TTG
        CCC GTT ACG CTT GTT TGT GCT GGC AAT CGA AGG AAA GAA CAG
        AAC ATG GTT AAA CAA ACC ATT GGT TTC AAC TGG GGC GCC GCT
        GCC GTT TCA ACA ACG ATA TGG CGC GGG GTA CCC CTC CGC GCT
        TTG CTA AAA CGG TGC GGT GTT TTT AGC AAG AAT AAA GGG GCG
        CTT AAT GTT TGC TTC GAA GGA GCT GAT GTG TTG CCC GGA GGT
        GGT GGT TCA AAG TAT GGA ACC AGC ATT AAG AAG GAA TTT GCA
        ATG GAT CCA GCA CGA GAT ATC ATC GTA GCC TAC ATG CAG AAC
        GGA GAA AAA TTG GCA CCC GAC CAC GGG TTT CCA GTA CGA ATG
        ATA ATT CCA GGA TTC ATT GGA GGA AGA ATG GTG AAA TGG ATA
        AAG AGG ATT ATA GTC ACC ACC CAA GAA TCA GAC AGC TAT TAT
        CAT TTC AAG GAC AAT AGA GTT CTT CCT CCC CAT GTT GAT GCT
        GAA CTT GCA AAT ACC GAA GCA TGG TGG TAC AAG CCA GAG TAT
        ATC ATC AAT GAG CTT AAT ATT AAC TCT GTC ATT ACG ACG CCG
        TGT CAT GAA GAA ATT TTG CCA ATT AAC GCC TGG ACG ACT CAG
        CGA CCT TAC ACG TTG AGG GGC TAT TCT TAT TCT GGC GGA GGG
        AAA AAA GTA ACG CGA GTA GAA GTG ACG TTG GAT GGA GGA GAA
        ACA TGG CAA GTT AGC ACA CTA GAT CAC CCA GAG AAG CCC ACC
        AAA TAT GGC AAG TAC TGG TGT TGG TGC TTT TGG TCA CTC GAG
        GTT GAG GTG TTA GAC TTG CTC AGT GCT AAA GAA ATT GCT GTT
        CGA GCT TGG GAT GAG ACC CTC AAT ACT CAA CCC GAG AAG CTT
```

EP 0 283 338 B1

```
ATT TGG AAC GTC ATG GGA ATG ATG AAT AAT TGC TGG TTC CGA
GTA AAG ATG AAT GTG TGC AAG CCT CAC AAG GGA GAG ATT GGA
ATA GTG TTT GAG CAT CCG ACT CAA CCT GGA AAC CAA TCA GGT
GGA TGG ATG GCG AAG GAG AGA CAT TTG GAG ATA TCA GCA GAG
GCA CCT CAA ACA CTA AAG AAG AGT ATC TCA ACT CCA TTC ATG
AAC ACA GCT TCC AAG ATG TAC TCC ATG TCC GAG GTC AGG AAA
CAC AGC TCT GCT GAC TCT GCT TGG ATC ATA GTC CAT GGT CAT
ATC TAT GAC GCC ACG CGT TTC TTG AAA GAT CAC CCT GGT GGG
ACT GAC AGC ATT CTC ATC AAT GCT GGC ACT GAT TGC ACT GAG
GAA TTT GAT GCA ATT CAT TCT GAT AAG GCT AAG AAG CTC TTG
GAG GAT TTC AGG ATT GGT GAA CTC ATA ACT ACT GGT TAC ACC
TCT GAC TCT CCT GGC AAC TCC GTG CAC GGA TCT TCT TCC TTC
AGC AGC TTT CTA GCA CCT ATT AAG GAA CTT GTT CCA GCG CAG
AGG AGT GTG GCC CTA ATT CCA AGA GAG AAA ATC CCA TGC AAA
CTC ATC GAC AAG CAA TCC ATC TCC CAT GAT GTT AGG AAA TTT
CGA TTT GCA TTG CCC TCT GAG GAT CAA GTC TTG GGC TTG CCT
GTT GGA AAA CAT ATC TTC CTC TGT GCC GTT ATT GAC GAT AAG
CTC TGC ATG CGC GCT TAC ACG CCT ACT AGC ACG ATC GAT GAG
GTG GGG TAC TTC GAG TTG GTT GTC AAG ATA TAC TTC AAA GGA
ATT CAC CCT AAA TTC CCC AAT GGA GGG CAA ATG TCA CAG TAT
CTT GAT TCT ATG CCG TTA GGG TCA TTT CTC GAC GTG AAA GGT
CCA TTA GGT CAC ATT GAA TAC CAA GGA AAG GGA AAT TTC TTA
GTT CAT GGC AAA CAG AAG TTT GCC AAG AAG TTG GCC ATG ATA
GCA GGT GGA ACA GGA ATA ACT CCA GTG TAT CAA GTC ATG CAG
GCA ATT CTG AAA GAT CCA GAA GAT GAC ACA GAA ATG TAT GTG
GTG TAT GCT AAC AGA ACA GAG GAT GAT ATT TTA CTT AAG GAA
GAG CTT GAT TCA TGG GCT GAG AAA ATT CCA GAG AGG GTT AAA
GTT TGG TAT GTG GTT CAG GAT TCT ATT AAA GAA GGA TGG AAG
TAC AGC ATT GGT TTT ATT ACA GAA GCC ATT TTG AGA GAA CAT
ATC CCT GAG CCA TCT CAC ACA ACA CTG GCT TTG GCT TGT GGA
CCA CCT CCT ATG ATT CAA TTT GCT GTT AAT CCA AAC TTG GAG
AAG ATG GGC TAT GAC ATT AAG GAT TCC TTA TTG GTG TTC TAA
```

33

FIG_1

FIG_2

λgt 11

λ13 29

λ13 34

λ13 28

_____ 2 kb

o    Eco RI
▲    Hind III
▼    Kpn I
▽    Sst I
△    Xmn I
◄    Pz
■    Lac Z

_____ 200 bp

FIG-3

1  2  3  4  5  6  7  8  9

−200 kd

−116 kd
−96 kd

−45 kd

+   +   −   +   ++

Coomassie      Western blot

FIG_4

FIG_5

FIG_6

**3 2 1**

−6 kb
−3 kb
−2 kb
−1 kb

# FIG.7

EP 0 283 338 B1

atcggc CAA TTC CCT TGT AGA GAA TTG CCC GTT ACG CTT GTT TGT GCT GGC AAT CGA AGG AAA GAA CAG AAC ATG GTT AAA CAA ACC ATT 90
  Glu Phe Pro Cys Arg Glu Leu Pro Val Thr Leu Val Cys Ala Gly Asn Arg Arg Lys Glu Gln Asn Met Val Lys Gln Thr Ile 28

GGT TTC AAC TGG GGC GCC GCT GCC GTT TCA ACA ACG ATA TGG CGC GGG GTA CCC CTC CGC GCT TTG CTA AAA CGG TGC GGT GTT TTT AGC 180
Gly Phe Asn Trp Gly Ala Ala Ala Val Ser Thr Thr Ile Trp Arg Gly Val Pro Leu Arg Ala Leu Leu Lys Arg Cys Gly Val Phe Ser 58

AAG AAT AAA GGC GCC CTT AAT GTT TGC TTC GAA GGA GCT GAT GTC TTG CCC GGA GGT GGT GGT TCA AAG TAT GGA ACC AGC ATT AAG AAG 270
Lys Asn Lys Gly Ala Leu Asn Val Cys Phe Glu Gly Ala Asp Val Leu Pro Gly Gly Gly Gly Ser Lys Tyr Gly Thr Ser Ile Lys Lys 88

GAA TTT GCA ATG GAT CCA GCA CCA GAT ATC ATC GTA GCC TAC ATC GAC AAC GGA GAA AAA TTG GCA CCC GAC CAC GCG TTT CCA GTA CGA 360
Glu Phe Ala Met Asp Pro Ala Arg Asp Ile Ile Val Ala Tyr Met Gln Asn Gly Glu Lys Leu Ala Pro Asp His Gly Phe Pro Val Arg 118

ATG ATA ATT CCA GGA TTC ATT GGA GGA AGA ATG GTC AAA TGG ATA AAG AGG ATT ATA GTC ACC ACC CAA GAA TCA GAC AGC TAT TAT CAT 450
Met Ile Ile Pro Gly Phe Ile Gly Gly Arg Met Val Lys Trp Ile Lys Arg Ile Ile Val Thr Thr Gln Glu Ser Asp Ser Tyr Tyr His 148

TTC AAG GAC AAT AGA GTT CTT CCT CCC CAT GTT GAT GCT GAA CTT GCA AAT ACC GAA GCA TGG TGG TAC AAG CCA GAG TAT ATC ATC AAT 540
Phe Lys Asp Asn Arg Val Leu Pro Pro His Val Asp Ala Glu Leu Ala Asn Thr Glu Ala Trp Trp Tyr Lys Pro Glu Tyr Ile Ile Asn 178

GAG CTT AAT ATT AAC TCT GTC ATT ACC ACG CCG TGT CAT GAA GAA ATT TTG CCA ATT AAC GCC TGG ACG ACT CAG CGA CCT TAC ACG TTG 630
Glu Leu Asn Ile Asn Ser Val Ile Thr Thr Pro Cys His Glu Glu Ile Leu Pro Ile Asn Ala Trp Thr Thr Gln Arg Pro Tyr Thr Leu 208

AGG GGC TAT TCT TAT TCT GGC GGA GGG AAA AAA GTA ACG CGA GTA GAA GTC ACC TTG GAT GGA GGA GAA ACA TGG CAA GTT AGC ACA CTA 720
Arg Gly Tyr Ser Tyr Ser Gly Gly Gly Lys Lys Val Thr Arg Val Glu Val Thr Leu Asp Gly Gly Glu Thr Trp Gln Val Ser Thr Leu 238

GAT CAC CCA GAG AAG CCC ACC AAA TAT GGC AAG TAC TGG TGT TGG TGC TTT TGG TCA CTC GAG GTT GAG GTG TTA GAC TTG CTC AGT GCT 810
Asp His Pro Glu Lys Pro Thr Lys Tyr Gly Lys Tyr Trp Cys Trp Cys Phe Trp Ser Leu Glu Val Glu Val Leu Asp Leu Leu Ser Ala 268

AAA GAA ATT GCT GTT CGA GCT TGG GAT GAG ACC CTC AAT ACT CAA CCC GAG AAG CTT ATT TGG AAC GTC ATG GGA ATG ATG AAT AAT TGC 900
Lys Glu Ile Ala Val Arg Ala Trp Asp Glu Thr Leu Asn Thr Gln Pro Glu Lys Leu Ile Trp Asn Val Met Gly Met Met Asn Asn Cys 298

Figure 8a

41

EP 0 283 338 B1

**Figure 8b**

```
TCG TTC CGA CTA AAG ATC AAT CTC TCC AAG CCT CAC AAG GGA GAG ATT GGA ATA GTG TTT GAG CAT CCG ACT CAA CCT GGA AAC CAA TCA    990
Trp Phe Arg Val Lys Met Asn Val Cys Lys Pro His Lys Gly Glu Ile Gly Ile Val Phe Glu His Pro Thr Gln Pro Gly Asn Gln Ser    328

GGT CCA TCC ATC CCG AAC GAG AGA CAT TTG GAG ATA TCA GCA GAG GCA CCT CAA ACA CTA AAG AAG AGT ATC TCA ACT CCA TTC ATG AAC    1080
Gly Gly Trp Met Ala Lys Glu Arg His Leu Glu Ile Ser Ala Glu Ala Pro Gln Thr Leu Lys Lys Ser Ile Ser Thr Pro Phe Met Asn    358

ACA GCT TCC AAG ATC TAC TCC ATC TCC GAG GTC ACG AAA CAC AGC TCT GCT GAC TCT GCT TGG ATC ATA GTC CAT GGT CAT ATC TAT GAC    1170
Thr Ala Ser Lys Met Tyr Ser Met Ser Glu Val Arg Lys His Ser Ser Ala Asp Ser Ala Trp Ile Ile Val His Gly His Ile Tyr Asp    388

GCC ACC CCT TTC TTG AAA GAT CAC CCT GGT GGG ACT GAC AGC ATT CTC ATC AAT GCT GGC ACT GAT TGC ACT GAG GAA TTT GAT GCA ATT    1260
Ala Thr Arg Phe Leu Lys Asp His Pro Gly Gly Thr Asp Ser Ile Leu Ile Asn Ala Gly Thr Asp Cys Thr Glu Glu Phe Asp Ala Ile    418

CAT TCT GAT AAC GCT AAG AAC CTC TTG GAG GAT TTC ACG ATT GGT GAA CTC ATA ACT ACT GGT TAC ACC TCT GAC TCT CCT GGC AAC TCC    1350
His Ser Asp Lys Ala Lys Lys Leu Leu Glu Asp Phe Arg Ile Gly Glu Leu Ile Thr Thr Gly Tyr Thr Ser Asp Ser Pro Gly Asn Ser    448

GTC CAC GGA TCT TCT TCC TTC AGC AGC TTT CTA GCA CCT ATT AAG GAA CTT GTT CCA GCG CAG AGG AGT GTC GCC CTA ATT CCA AGA GAG    1440
Val His Gly Ser Ser Ser Phe Ser Ser Phe Leu Ala Pro Ile Lys Glu Leu Val Pro Ala Gln Arg Ser Val Ala Leu Ile Pro Arg Glu    478

AAA ATC CCA TGC AAA CTC ATC GAC AAG CAA TCC ATC TCC CAT GAT GTT ACG AAA TTT CGA TTT GCA TTG CCC TCT GAG GAT CAA GTC TTG    1530
Lys Ile Pro Cys Lys Leu Ile Asp Lys Gln Ser Ile Ser His Asp Val Arg Lys Phe Arg Phe Ala Leu Pro Ser Glu Asp Gln Val Leu    508

GGC TTC CCT GTT GGA AAA CAT ATC TTC CTC TCT GCC GTT ATT GAC GAT AAG CTC TGC ATG CGC GCT TAC ACG CCT ACT AGC ACG ATC GAT    1620
Gly Leu Pro Val Gly Lys His Ile Phe Leu Cys Ala Val Ile Asp Asp Lys Leu Cys Met Arg Ala Tyr Thr Pro Thr Ser Thr Ile Asp    538

GAG CTC GGG TAC TTC GAG TTG GTT GTC AAG ATA TAC TTC AAA GGA ATT cca                                                         1671
Glu Val Gly Tyr Phe Glu Leu Val Val Lys Ile Tyr Phe Lys Gly Ile                                                            554
```

42

Figure 9a

Figure 9 b

Figure 10

```
MetAlaAlaSerValGluAsnArgGlnPheSerHisLeuGluAlaGlyLeuSerArgSerPheLysProArgSer
ATGGCGGCATCTGTCGAAAACAGGCAGTTCAGTCACCTAGAAGCCGGTTTATCCCGGTCTTTCAAGCCCCGGTCT

AspSerProValArgGlyCysAsnPheProSerProAsnSerThrAsnPheGlnLysLysProAsnSerThrIle
GATTCCCCGGTTCGTGGCTGCAACTTCCCTTCGCCCAACAGTACTAATTTCCAAAAGAAACCAAATTCCACCATT

TyrLeuAspTyrSerSerSerGluAspAspAspAspAspGluLysAsnGluTyrLeuGlnMetIleLysLys
TACCTTGATTACTCGTCGAGTGAAGACGACGATGATGATGACGAAAAAAATGAGTACCTTCAAATGATTAAAAAA

GlyAsnSerGluLeuGluProSerValHisAspThrArgAspGluGlyThrAlaAspAsnTrpIleGluArgAsn
GGGAATTCAGAGTTAGAGCCATCTGTTCATGACACTAGGGACGAAGGTACCGCTGATAATTGGATTGAACGCAAC

PheSerMetIleArgLeuThrGlyLysHisProPheAsnSerGluProProLeuAsnArgLeuMetHisHisGly
TTTTCCATGATTCGTCTCACCGGAAAGCATCCATTTAACTCCGAACCACCGTTGAACCGGCTCATGCACCACGGC

PheIleThrProValProLeuHisTyrValArgAsnHisGlyProValProLysGlyThrTrpAspAspTrpThr
TTTATCACACCGGTCCCACTTCATTACGTTCGTAACCATGGACCGGTTCCCAAGGGCACGTGGGATGACTGGACC

ValGluValThrGlyLeuValLysArgProMetLysPheThrMetAspGlnLeuValAsnGluPheProCysArg
GTGGAAGTCACGGGACTAGTGAAGCGTCCTATGAAATTCACAATGGACCAGTTGGTTAACGAATTCCCTTGTAGA

GluLeuProValThrLeuValCysAlaGlyAsnArgArgLysGluGlnAsnMetValLysGlnThrIleGlyPhe
GAATTGCCCGTTACGCTTGTTTGTGCTGGCAATCGAAGGAAAGAACAGAACATGGTTAAACAAACCATTGGTTTC

AsnTrpGlyAlaAlaAlaValSerThrThrIleTrpArgGlyValProLeuArgAlaLeuLeuLysArgCysGly
AACTGGGGCGCCGCTGCCGTTTCAACAACGATATGGCGCGGGGTACCCCTCCGCGCTTTGCTAAAACGGTGCGGT

ValPheSerLysAsnLysGlyAlaLeuAsnValCysPheGluGlyAlaAspValLeuProGlyGlyGlyGlySer
GTTTTTAGCAAGAATAAAGGGGCGCTTAATGTTTGCTTCGAAGGAGCTGATCTGTTGCCCGGAGGTGGTGGTTCA

LysTyrGlyThrSerIleLysLysGluPheAlaMetAspProAlaArgAspIleIleValAlaTyrMetGlnAsn
AAGTATGGAACCAGCATTAAGAAGGAATTTGCAATGGATCCAGCACGAGATATCATCGTAGCCTACATGCAGAAC

GlyGluLysLeuAlaProAspHisGlyPheProValArgMetIleIleProGlyPheIleGlyGlyArgMetVal
GGAGAAAAATTGGCACCCGACCACGGGTTTCCAGTACGAATGATAATTCCAGGATTCATTGGAGGAAGAATGGTG

LysTrpIleLysArgIleIleValThrThrGlnGluSerAspSerTyrTyrHisPheLysAspAsnArgValLeu
AAATGGATAAAGAGGATTATAGTCACCACCCAAGAATCAGACAGCTATTATCATTTCAAGGACAATAGAGTTCTT

ProProHisValAspAlaGluLeuAlaAsnThrGluAlaTrpTrpTyrLysProGluTyrIleIleAsnGluLeu
CCTCCCCATGTTGATGCTGAACTTGCAAATACCGAAGCATGGTGGTACAAGCCAGAGTATATCATCAATGAGCTT

AsnIleAsnSerValIleThrThrProCysHisGluGluIleLeuProIleAsnAlaTrpThrThrGlnArgPro
AATATTAACTCTGTCATTACGACGCCGTGTCATGAAGAAATTTTGCCAATTAACGCCTGGACGACTCAGCGACCT

TyrThrLeuArgGlyTyrSerTyrSerGlyGlyGlyLysValThrArgValGluValThrLeuAspGlyGly
TACACGTTGAGGGGCTATTCTTATTCTGGCGGAGGGAAAAAAGTAACGCGAGTAGAAGTGACGTTGGATGGAGGA

GluThrTrpGlnValSerThrLeuAspHisProGluLysProThrLysTyrGlyLysTyrTrpCysTrpCysPhe
GAAACATGGCAAGTTAGCACACTAGATCACCCAGAGAAGCCCACCAAATATGGCAAGTACTGGTGTTGGTGCTTT

TrpSerLeuGluValGluValLeuAspLeuLeuSerAlaLysGluIleAlaValArgAlaTrpAspGluThrLeu
TGGTCACTCGAGGTTGAGGTGTTAGACTTGCTCAGTGCTAAAGAAATTGCTGTTCGAGCTTGGGATGAGACCCTC

AsnThrGlnProGluLysLeuIleTrpAsnValMetGlyMetMetAsnAsnCysTrpPheArgValLysMetAsn
AATACTCAACCCGAGAAGCTTATTTGGAACGTCATGGGAATGATGAATAATTGCTGGTTCCGAGTAAAGATGAAT

ValCysLysProHisLysGlyGluIleGlyIleValPheGluHisProThrGlnProGlyAsnGlnSerGlyGly
GTGTGCAAGCCTCACAAGGGAGAGATTGGAATAGTGTTTGAGCATCCGACTCAACCTGGAAACCAATCAGGTGGA
```

45

```
    TrpMetAlaLysGluArgHisLeuGluIleSerAlaGluAlaProGlnThrLeuLysLysSerIleSerThrPro
    TGGATGGCGAAGGAGAGACATTTGGAGATATCAGCAGAGGCACCTCAAACACTAAAGAAGAGTATCTCAACTCCA
1501      1511      1521      1531      1541      1551      1561
    PheMetAsnThrAlaSerLysMetTyrSerMetSerGluValArgLysHisSerSerAlaAspSerAlaTrpIle
    TTCATGAACACAGCCTTCCAAGATGTACTCCATGTCCGAGGTCAGGAAACACAGCTCTGCTGACTCTGCTTGGATC
1576      1586      1596      1606      1616      1626      1636
    IleValHisGlyHisIleTyrAspAlaThrArgPheLeuLysAspHisProGlyGlyThrAspSerIleLeuIle
    ATAGTCCATGGTCATATCTATGACGCCACGCGTTTCTTGAAAGATCACCCTGGTGGGACTGACAGCATTCTCATC
1651      1661      1671      1681      1691      1701      1711
    AsnAlaGlyThrAspCysThrGluGluPheAspAlaIleHisSerAspLysAlaLysLysLeuLeuGluAspPhe
    AATGCTGGGACTGATTGCACTGAGGAATTTGATGCAATTCATTCTGATAAGGCTAAGAAGCTCTTGGAGGATTTC
1726      1736      1746      1756      1766      1776      1786
    ArgIleGlyGluLeuIleThrThrGlyTyrThrSerAspSerProGlyAsnSerValHisGlySerSerSerPe
    AGGATTGGTGAACTCATAACTACTGGTTACACCTCTGACTCTCCTGGCAACTCCGTGCACGGATCTTCTTCCTTC
1801      1811      1821      1831      1841      1851      1861
    SerSerPheLeuAlaProIleLysGluLeuValProAlaGlnArgSerValAlaLeuIleProArgGluLysIle
    AGCAGCTTTCTAGCACCTATTAAGGAACTTGTTCCAGCGCAGAGGAGTGTGGCCCTAATTCCAAGAGAGAAAATC
1876      1886      1896      1906      1916      1926      1936
    ProCysLysLeuIleAspLysGlnSerIleSerHisAspValArgLysPheArgPheAlaLeuProSerGluAsp
    CCATGCAAACTCATCGACAAGCAATCCATCTCCCATGATGTTAGGAAATTTCGATTTGCATTGCCCTCTGAGGAT
1951      1961      1971      1981      1991      2001      2011
    GlnValLeuGlyLeuProValGlyLysHisIlePheLeuCysAlaValIleAspAspAspLysLeuCysMetArgAla
    CAAGTCTTGGGCTTGCCTGTTGGAAAACATATCTTCCTCTGTGCCGTTATTGACGATAAGCTCTGCATGCGCGCT
2026      2036      2046      2056      2066      2076      2086
    TyrThrProThrSerThrIleAspGluValGlyTyrPheGluLeuValValLysIleTyrPheLysGlyIleHis
    TACACGCCTACTAGCACGATCGATGAGGTGGGGTACTTCGAGTTGGTTGTCAAGATATACTTCAAAGGAATTCAC
2101      2111      2121      2131      2141      2151      2161
    ProLysPheProAsnGlyGlyGlnMetSerGlnTyrLeuAspSerMetProLeuGlySerPheLeuAspValLys
    CCTAAATTCCCCAATGGGAGGGCAAATGTCACAGTATCTTGATTCTATGCCGTTAGGGTCATTTCTCGACGTGAAA
2176      2186      2196      2206      2216      2226      2236
    GlyProLeuGlyHisIleGluTyrGlnGlyLysGlyAsnPheLeuValHisGlyLysGlnLysPheAlaLysLys
    GGTCCATTAGGTCACATTGAATACCAAGGAAAGGGAAATTTCTTAGTTCATGGCAAACAGAAGTTTGCCAAGAAG
2251      2261      2271      2281      2291      2301      2311
    LeuAlaMetIleAlaGlyGlyThrGlyIleThrProValTyrGlnValMetGlnAlaIleLeuLysAspProGlu
    TTGGCCATGATAGCAGGTGGAACAGGAATAACTCCAGTGTATCAAGTCATGCAGGCAATTCTGAAAGATCCAGAA
2326      2336      2346      2356      2366      2376      2386
    AspAspThrGluMetTyrValValTyrAlaAsnArgThrGluAspAspIleLeuLeuLysGluGluLeuAspSer
    GATGACACAGAAATGTATGTGGTGTATGCTAACAGAACAGAGGATGATATTTTACTTAAGGAAGAGCTTGATTCA
2401      2411      2421      2431      2441      2451      2461
    TrpAlaGluLysIleProGluArgValLysValTrpTyrValValGlnAspSerIleLysGluGlyTrpLysTyr
    TGGGCTGAGAAAATTCCAGAGAGGGTTAAAGTTTGGTATGTGGTTCAGGATTCTATTAAAGAAGGATGGAAGTAC
2476      2486      2496      2506      2516      2526      2536
    SerIleGlyPheIleThrGluAlaIleLeuArgGluHisIleProGluProSerHisThrThrLeuAlaLeuAla
    AGCATTGGTTTTATTACAGAAGCCATTTTGAGAGAACATATCCCTGAGCCATCTCACACAACACTGGCTTTGGCT
2551      2561      2571      2581      2591      2601      2611
    CysGlyProProProMetIleGlnPheAlaValAsnProAsnLeuGluLysMetGlyTyrAspIleLysAspSer
    TGTGGACCACCTCCTATGATTCAATTTGCTGTTAATCCAAACTTGGAGAAGATGGGCTATGACATTAAGGATTCC
2626      2636      2646      2656      2666      2676      2686
    LeuLeuValPhe***
    TTATTGGTGTTCTAA
2701      2711
```